# EUROPEAN PATENT APPLICATION

(11) **EP 4 714 949 A1**
(43) Date of publication of application: **25.03.2026**
(21) Application number: 24807592.1
(22) Date of filing: 17.05.2024
(51) Int. Cl.: C07D 401/14, A61K 31/496, A61P 19/00

(54) **NOVEL COMPOUNDS CAPABLE OF INDUCING OSTEOGENIC DIFFERENTIATION**

(30) Priority: 18.05.2023 KR 20230064583
(71) Applicant: AJOU UNIVERSITY INDUSTRY-ACADEMIC COOPERATION FOUNDATION, Gyeonggi-do 16499 (KR); Seoul National University Dental Hospital, Seoul 03080 (KR)
(72) Inventor: CHOI, Junwon, Suwon-si Gyeonggi-do 16499 (KR); KIM, Jin Man, Seoul 05268 (KR)
(74) Representative: Mewburn Ellis LLP
(86) International application number: PCT/KR2024/006699
(87) International publication number: WO 2024/237721

(57) **Abstract**

The present invention relates to a novel compound capable of inducing osteogenic differentiation of stem cells and, more particularly, to a compound of chemical formula (I), a stereoisomer thereof or a pharmaceutically acceptable salt thereof, and a composition for inducing osteogenic differentiation of stem cells, comprising the compound, the stereoisomer thereof or the pharmaceutically acceptable salt thereof. The novel compound based on nutlin, according to the present invention, induces the degradation of MDM2 to efficiently promote the differentiation of mesenchymal stromal cells, and thus, the effect of inducing osteogenic differentiation of stem cells is excellent.

## Description

### [Technical Field]

The present invention relates to a novel compound for inducing osteogenic differentiation of stem cells, and more specifically, to a compound of Formula (I), a stereoisomer thereof, or a pharmaceutically acceptable salt thereof, and a composition for inducing osteogenic differentiation of stem cells containing the compound, the stereoisomer thereof, or the pharmaceutically acceptable salt thereof.

### [Background Art]

In the pathway where proteins are degraded by the proteasome, which is involved in intracellular protein degradation, the proteins are tagged with ubiquitin (Ub) and then degraded by the proteasome in the cytoplasm and nucleus. This ubiquitin-mediated protein degradation regulatory system functions to maintain cellular homeostasis by removing damaged or unnecessary proteins.

Ubiquitin, which is a protein containing 76 amino acids, is regulated by a cascade of E3 (ubiquitin ligase enzymes). The E3 that consists of a binding domain and a substrate recognition domain brings the E2-Ub complex into close proximity with the substrate protein, to facilitate the transfer of Ub from the E2-Ub to the lysine residue of the substrate protein. Ub may be conjugated to one or more lysine residues on substrate proteins, or form polyubiquitine (poly-Ub) chains in which multiple Ub molecules are linked into ubiquitin chains. In particular, lysine-48-linked polyubiquitinated proteins are delivered to the ATP-dependent proteolytic complex 26S proteasome, where unfolding and degradation occur. This process is referred to as the ubiquitin-proteasome system (UPS). 600 or more different types of E3 in the UPS are known (Kleiger, G., and Mayor, T. (2014). Perilous Journey: a Tour of the Ubiquitin-Proteasome System. Trends Cel Biol. 24, 352-359).

E3 ligases are classified into RING (really interesting new gene)-type E3 ligases, HECT (homologous to the E6AP carboxyl terminus)-type E3 ligases, RBR (RING-between-RING)-type E3 ligases, and U-box-type E3 ligases based on the presence of characteristic domain structures and differences in the Ub transfer mechanisms. Among these, U-box-type and RING-type E3 ligases are the most abundant types, which have a U-box domain and a RING domain, respectively, and thus serve as anchors for the E2-Ub complex. The U-box and RING domains directly bind to E2-Ub to provide proximity between the Ub of the E2-Ub complex and the amino group of the substrate protein and stimulate catalytic activity by modulating conformational changes beyond the binding site to control the rate of ubiquitination of adjacent substrate proteins.

E3 binds to both E2-Ub and substrate proteins, providing specificity for recognizing substrate proteins to be ubiquitinated. In other words, polyubiquitination of substrate proteins is determined by E3 enzymes, and all substrate proteins have a recognition site for a specific E3 and a Ub linkage site where ubiquitination occurs. Considering the cellular environment, where approximately 100,000 proteins exist, each E3 has substrate specificity, and also redundancy and multiplicity.

Proteolysis-targeting chimera (PROTAC) has been developed to induce protein degradation via the UPS by polyubiquitination of target proteins through such E3.

In the PROTAC, when small molecules that specifically recognize target proteins and small molecules that specifically recognize E3 recognize E3 and binds to the E3, the E3 and the target protein become close to each other, inducing E3-mediated polyubiquitination of the target protein. Induction of polyubiquitination within the cell ultimately leads to the recognition of the polypolyubiquitinized target protein by the 26S proteasome, an ATP-dependent protease complex, causing degradation via the unregulated protein transporter (UPS).

Under this technical background, the present inventors have developed a novel compound targeting MDM2 based on nutlin containing: an E3 ligand which is an E3-binding module that specifically recognizes an E3; an MDM2 (mouse double minute 2 homolog) ligand, which is an E3-binding module that specifically recognizes a target protein; and a linker and found that the compound has the ability to induce osteogenic differentiation of stem cells, thereby completing the present invention.

### [Disclosure]

It is one object of the present invention to provide a novel compound for inducing osteogenic differentiation of stem cells, a stereoisomer thereof, or a pharmaceutically acceptable salt thereof.

It is another aspect of the present invention to provide a composition for inducing osteogenic differentiation of stem cells containing the compound, the stereoisomer thereof, or the pharmaceutically acceptable salt thereof.

It is another aspect of the present invention to provide a method of inducing osteogenic differentiation of stem cells using the compound, the stereoisomer thereof, or the pharmaceutically acceptable salt thereof.

It is another aspect of the present invention to provide the use of the compound, stereoisomer thereof, or the pharmaceutically acceptable salt thereof for inducing osteogenic differentiation of stem cells.

It is another aspect of the present invention to provide the use of the compound, the stereoisomer thereof, or the pharmaceutically acceptable salt thereof for preparing a composition for inducing osteogenic differentiation of stem cells.

### [Technical Solution]

In accordance with the present invention, the above and other objects can be accomplished by the provision of a compound of Formula (I), a stereoisomer thereof, or a pharmaceutically acceptable salt thereof: 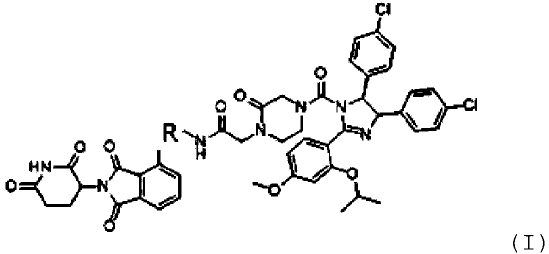
wherein R is 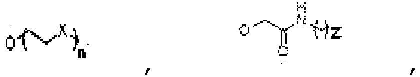 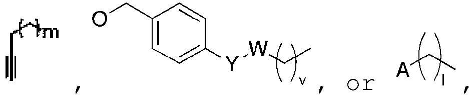
X is C, O, or N, n is an integer of 1 to 5,
Z is an integer of 1 to 10,
m is an integer of 1 to 10,
Y-W is C≡C, or both Y and W are C, or Y is C and W is O,
v is an integer of 1 to 4,
A is C or N, and
l is an integer of 1 to 8.

In accordance with another aspect of the present invention, there is provided a composition for inducing osteogenic differentiation of stem cells containing comprising the compound, the stereoisomer thereof, or the pharmaceutically acceptable salt thereof.

In accordance with another aspect of the present invention, there is provided a method of inducing osteogenic differentiation of stem cells using the compound, the stereoisomer thereof, or the pharmaceutically acceptable salt thereof.

In accordance with another aspect of the present invention, there is provided the use of the compound, the stereoisomer thereof, or the pharmaceutically acceptable salt thereof for inducing osteogenic differentiation of stem cells.

In accordance with another aspect of the present invention, there is provided the use of the compound, the stereoisomer thereof, or the pharmaceutically acceptable salt thereof for preparing a composition for inducing osteogenic differentiation of stem cells.

### [Description of Drawings]

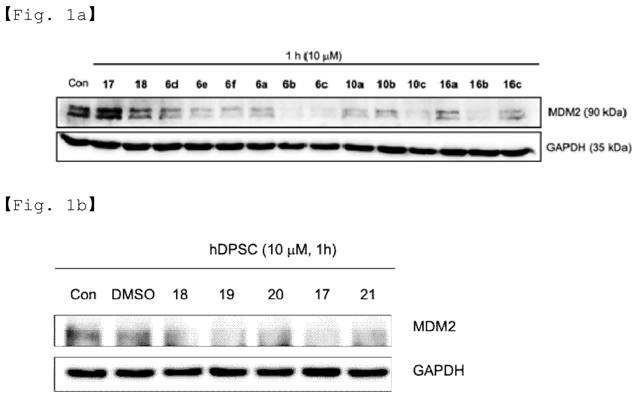

FIGs. 1A and 1B illustrate the MDM2 degradation effect of an MDM2-targeting PROTAC using an RBN ligand. In FIG. 1A, 17: tert-butyl 2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)acetate 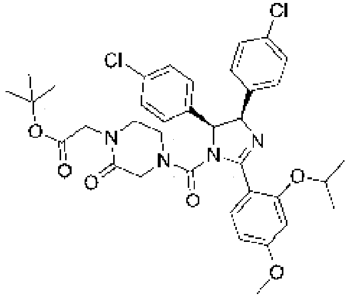 18: 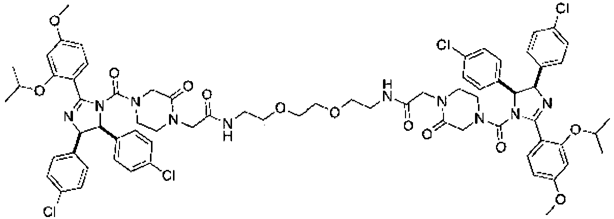
FIG. 2 illustrates the results of selection of two compounds that effectively degrade MDM2 and activate p53, and treatment concentrations.
FIG. 3A shows the biomineralization effect of the secondarily selected compounds confirmed by Alizarin Red S staining.
FIG. 3B shows the expression of osteogenic differentiation marker genes for the secondarily selected compounds.
FIG. 3C shows the biomineralization effect of compounds 17 to 21 confirmed by Alizarin Red S staining.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

In one aspect, the present invention is directed to a compound of Formula (I), a stereoisomer thereof, or a pharmaceutically acceptable salt thereof: 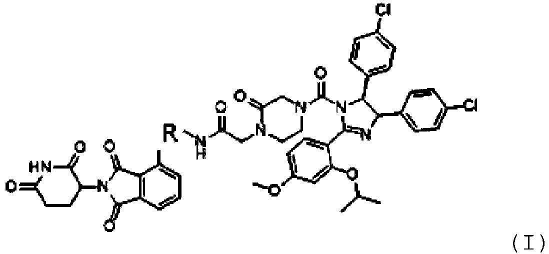
wherein R is 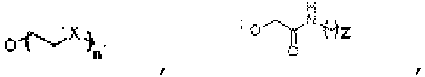 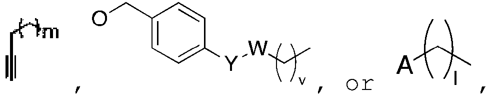
X is C, O, or N, n is an integer of 1 to 5,
Z is an integer of 1 to 10,
m is an integer of 1 to 10,
Y-W is C≡C, or both Y and W are C, or Y is C and W is O,
v is an integer of 1 to 4,
A is C or N, and
l is an integer of 1 to 8.

The term "alkyl" refers to a monovalent moiety obtained by removing a hydrogen atom from a carbon atom of an aliphatic or alicyclic, saturated or unsaturated (unsaturated, fully unsaturated) hydrocarbon compound. Examples of the saturated alkyl include methyl, ethyl, propyl, butyl, pentyl, hexyl, heptyl, and the like; examples of the saturated straight-chain alkyl include methyl, ethyl, n-propyl, n-butyl, n-pentyl(amyl), n-hexyl, n-heptyl, and the like; and examples of the saturated branched-chain alkyl include isopropyl, isobutyl, sec-butyl, tert-butyl, isopentyl, neopentyl, and the like.

The term "alkoxy" refers to -OR [wherein R is an alkyl group] and examples of the alkoxy include methoxy, ethoxy, n-propoxy, isopropoxy, n-butoxy, sec-butoxy, isobutoxy, tert-butoxy and the like.

The term "aryl" refers to a monovalent moiety obtained by removing a hydrogen atom from an aromatic ring atom in an aromatic compound.

The term "alkenyl" refers to an alkyl group with one or more carbon-carbon double bonds. Examples of unsaturated alkenyl groups include ethenyl (vinyl, - CH=CH₂), 1-propenyl (-CH=CHCH₃), 2-propenyl, isopropenyl, butenyl, pentenyl, hexenyl and the like.

As used herein, the term "alkynyl" refers to an alkyl group having one or more carbon-carbon triple bonds. Examples of unsaturated alkynyl groups include ethynyl and 2-propynyl.

As used herein, the term "formyl" includes - C(=O)H.

As used herein, the term "aryl" refers to a monovalent moiety obtained by removing a hydrogen atom from an aromatic ring atom of an aromatic compound. For example, the term "_{C5-7}aryl" refers to a monovalent moiety having 5 to 7 ring atoms, obtained by removing a hydrogen atom from an aromatic ring atom of an aromatic compound, and the term "_{C5-10}aryl" refers to a monovalent moiety having 5 to 10 ring atoms, obtained by removing a hydrogen atom from an aromatic ring atom of an aromatic compound. Here, the prefix (such as C₅₋₇ or C₅₋₁₀) refers to the number of ring atoms, regardless of whether the atoms are carbon atoms or heteroatoms. For example, the term "_{C5-6}aryl" refers to an aryl group having five or six ring atoms. Here, all of the ring atoms may be carbon atoms, like "carboaryl group". Examples of the carboaryl group include, but are not limited to, groups derived from benzene, naphthalene, azulene, anthracene, phenanthrene, naphthacene, and pyrene. Examples of the aryl group containing a fused ring, at least one of which is an aromatic ring, include, but are not limited to, groups derived from indane, indene, isoindene, tetralin, acenaphthene, fluorene, phenalene, acephenanthrene, and aceantrene. Alternatively, the ring atoms may include one or more heteroatoms, like "heteroaryl groups".

The term "heteroaryl" referes to an aryl containing one or more heteroatoms and examples thereof include pyridine, pyrimidine, benzothiophene, furyl, dioxalanyl, pyrrolyl, oxazolyl, pyridyl, pyridazinyl, pyrimidinyl, and the like, more specifically, C₉ having two fused rings derived from benzofuran, isobenzofuran, indole, isoindole, indolizine, indoline, isoindoline, purine (adenine or guanine), benzimidazole, indazole, benzoxazole, benzisoxazole, benzodioxole, benzofuran, benzotriazole, benzothiofuran, benzothiazole, benzothiadiazole, C₁₀ having two fused rings derived from chromene, isochromene, chromane, isochromane, benzodioxane, quinoline, isoquinoline, quinolizine, benzoxazine, benzodiazine, pyridopyridine, quinoxaline, quinazoline, cinnoline, phthalazine, naphthyridine, and pteridine; C₁₁ having two fused rings derived from benzodiazepine; C₁₃ having three fused rings derived from carbazole, dibenzofuran, dibenzothiophene, carboline, perimidine, and pyridoindole; and C₁₄ having three fused rings derived from acridine, xanthene, thioxanthene, oxantrene, phenoxathiine, phenazine, phenothiazine, thianthrene, phenanthridine, phenanthroline, and phenazine.

The term "heterocyclyl" refers to a monovalent moiety obtained by removing a hydrogen atom from a ring atom of a heterocyclic compound.

The term "prefix" (such as C₁₋₁₂ or C₁₋₈) refers to the number of ring atoms, regardless of whether the atoms are carbon atoms or heteroatoms. For example, the term "_{C3-6}heterocyclyl" as used herein refers to a heterocyclyl group having 3 to 6 ring atoms.

Examples of monocyclic heterocyclyl groups include, but are not limited to, those derived from:
N₁: aziridine, azetidine, pyrrolidine, pyrroline, 2H- or 3H-pyrrole, piperidine, dihydropyridine, tetrahydropyridine, azepine;
N₂: imidazolidine, pyrazolidine, imidazoline, pyrazoline, piperazine;
O₁: oxirane, oxetane, oxolane, oxole, oxane, dihydropyran, pyran, oxepin;
O₂: dioxolane, dioxane, and dioxepane;
O₃: trioxane;
N₁O₁: tetrahydrooxazole, dihydrooxazole, tetrahydroisoxazole, dihydroisoxazole, morpholine, tetrahydrooxazine, dihydrooxazine, oxazine
S₁: thiirane, thietane, thiolane, thiane, thiepane;
N₁S₁: thiazoline, thiazolidine, thiomorpholine;
N₂O₁: oxadiazine;
O₁S₁: oxathiol, oxathiane; and
N₁O₁S₁: oxathiazine.

In an embodiment, R is 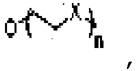 X is C or O, and n is 1 to 3.

Specifically, X is C and n is 1, X is C and n is 2, X is C and n is 3, X is O and n is 1, X is O and n is 2, or X is O and n is 3.

In a specific embodiment of the present invention, the compound may include one or more compounds selected from the group consisting of:
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(5-((2-(2,6dioxopiperidin-3-yl)-1,3-dioxoisoindolin-4-yl)oxy)pentyl) acetamide;
2-(4-(cis-4,5-bis(4-chlorophenyl)-2-(2-isopropoxy-4-methoxyphenyl)-4,5-dihydro-1H-imidazole-1-carbonyl)(2-(2,6-dioxo-opiperidin-3-yl)-1,3-dioxoisoindolin-4-yl)oxy)octyl) acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(11-((2-(2,6-dioxop-erydin-3-yl)-1,3-dioxoisoindolin-4-yl)oxy) undecyl) acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-((2-(2,6-dioxopiperidin-3-yl)-1,3-dioxoisoindolin-4-yl)oxy) ethoxy)ethyl)acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-(2-((2-(2,6-dioxo-opiperidin-3-yl))-1,3-dioxoisoindolin-) 4-yl)oxy)ethoxy)ethoxy)ethoyl)acetamide; and
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-(2-(2-((2-(2,6-dioxopiperidin-3-yl))-1,3-dioxo)isoindolin-4-yl)oxy))ethoxy)ethoxy)-ethoxy)ethyl)-acetamide.

The specific synthetic procedure and conditions for the compound are as follows:

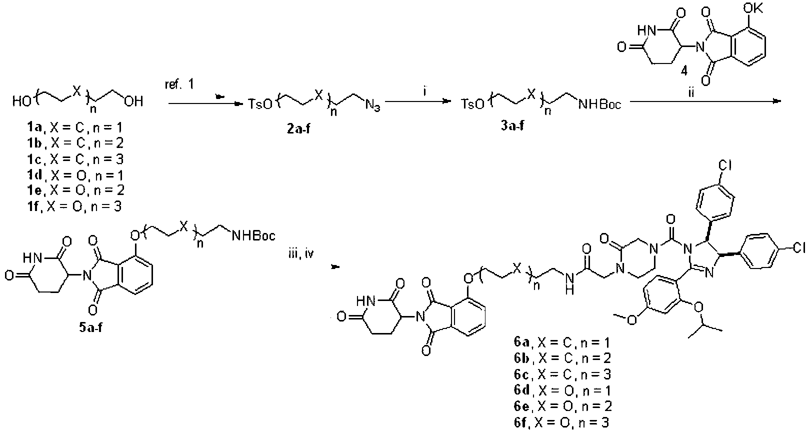

Reagents and conditions: (i) H₂, Pd/C, (Boc)₂O, THF, rt, 1 h; (ii) DIPEA, CH₃CN, 70°C, 16 h; (iii) TFA, CH₂Cl₂, rt, 1 h; (iv) nutlin acid, HATU, DIPEA, DMF, rt, 16 h.

In another embodiment, R is 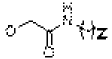 and Z is 2 to 8.

Specifically, R is 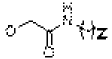 and Z is 2, or R is 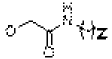 and Z is 5, or R is 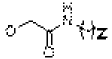 and Z is 5 or 8.

In a specific embodiment of the present invention, the compound may include one or more compounds selected from the group consisting of:
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-((2-(2,6-dioxopiperidin-3-yl)-1,3-dioxoisoindolin-4-yl))oxy)acetamide)ethyl)-acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(5-(2-((2-(2,6-dioxopiperidin-3-yl)-1,3-dioxoisoindolin-4-yl))oxy) acetamide)pentyl-l)acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(8-(2-((2-(2,6-dioxopiperidin-3-yl)-1,3-dioxoisoindolin-4-yl))oxy)acetamide)octyl)-acetamide.

The specific synthetic procedure and conditions for the compound are as follows:

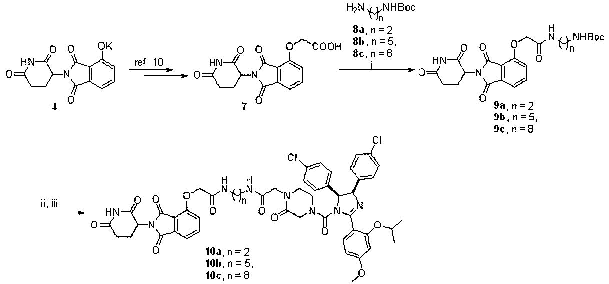

Reagents and conditions: (i) HATU, DIPEA, DMF, rt, 16 h; (ii) TFA, CH₂Cl₂, rt, 1 h; (iii) nutlinic acid, HATU, DIPEA, DMF, rt, 16 h.

In another embodiment, R is 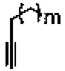 and M is 3 to 9. Specifically, R is 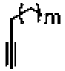 and m is 3, or R is 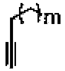 and m is 6, or R is 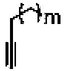 and m is 9.

In a specific embodiment of the present invention, the compound may include one or more compounds selected from the group consisting of:
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(6-(2-(2,6-dioxopipelysin-3-yl)-1,3-dioxoisoindolin-4-yl)hex-5-yn-1-yl) acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(9-(2-(2,6-dioxopiperidin-3-yl)-1,3-dioxoisoindolin-4-yl)non-8-yn-1-yl) acetamide; and
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(9-(2-(2,6-dioxopiperidin-3-yl)-1,3-dioxoisoindolin-4-yl)dodec-11-yn-1-yl) acetamide

The specific synthetic procedures and conditions for the compounds are as follows:

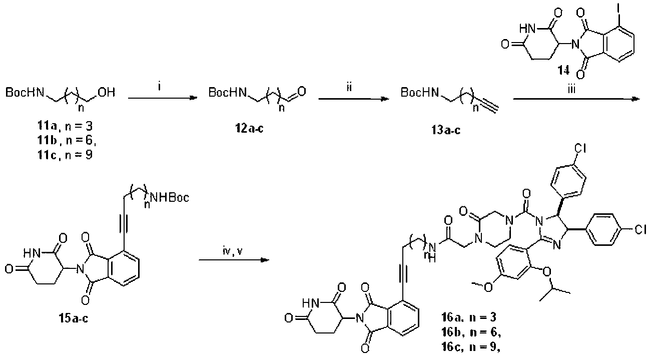

Reagents and conditions: (i) (COCl)₂, DMSO, DIPEA, CH₂Cl₂, -78 °C to -30°C, 1 h or PCC, Al₂O₃, CH₂Cl₂, rt, 3 h; (ii) Dimethyl (1-diazo-2-oxopropyl)phosphonate, K₂CO₃, CH₃OH, rt, 12 h; (iii) CuI, PdCl₂(PPh₃)₂, Et₃N, DMF, 80°C, 16 h; (iv) TFA, CH₂Cl₂, rt, 1 h; (v) nutlinic acid, HATU, DIPEA, DMF, rt, 16 h.

In another embodiment, R may 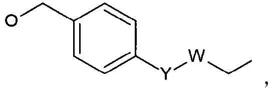 Y-W may be C≡C, or both Y and W may be C, or Y may be C and W may be O.

In a specific embodiment of the present invention, the compound may be:
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(8-((2-(2,6-dioxopiperidin-3-yl)-1,3-dioxoisoindolin-4-yl)amino)octyl)acetamide.

The specific synthetic process and conditions for the compound are as follows:

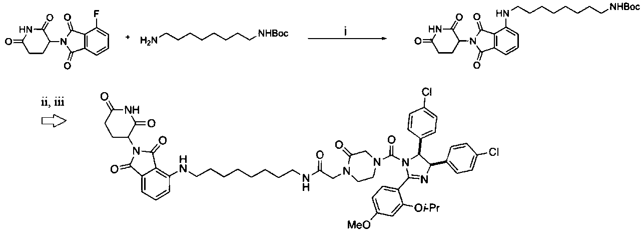

Reagents and conditions: (i) DIPEA, NMP, 90°C, 17 h; (ii) TFA, CH₂Cl₂, rt, 1 h; (iii) nutlinic acid, HATU, DIPEA, DMF, rt, 16 h.

In a specific embodiment of the present invention, the compound may be:
2-(4-(*cis*-4,5-bis(4-chlorophenyl)-2-(2-isopropoxy-4-methoxyphenyl)-4,5-dihydro-1H-imidazole-1-carbonyl)-2-oxopipera zin-1-yl)-N-(4-(4-(((2-(2,6-dioxopiperidin-3-yl)-1,3-dioxoisoindolin-4-yl)oxy)methyl)phenyl)but-3-yn-1-yl)acetamide.

The specific synthetic procedures and conditions for the compounds are as follows:

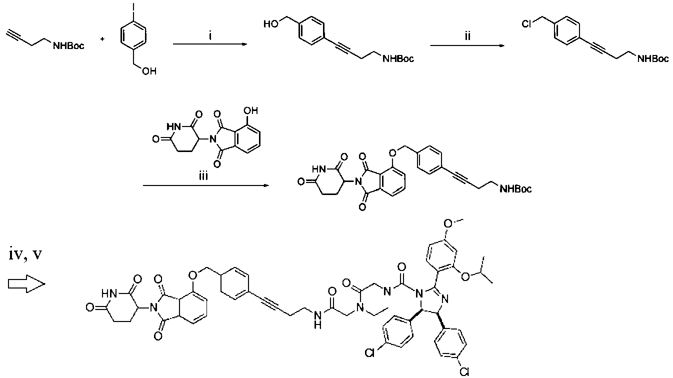

Reagents and conditions: (i) CuI, PdCl₂(PPh₃)₂, Et3N, DMF, 80°C, 3 h; (ii) MsCl, NEt3, CH₂Cl₂, 0°C to rt, 25 h; (iii) K₂CO₃, DMF, 60°C, 4 h; (iv) TFA, CH₂Cl₂, rt, 1 h; (v) nutlinic acid, HATU, DIPEA, DMF, rt, 16 h.

In a specific embodiment of the present invention, the compound may be:
2-(4-(*cis*-4,5-bis(4-chlorophenyl)-2-(2-isopropoxy-4-methoxyphenyl)-4,5-dihydro-1H-imidazole-1-carbonyl)-2-oxopi perazin-1-yl)-N-(4-(4-(((2-(2,6-dioxopiperidin-3-yl)-1,3-dioxoisoindolin-4-yl)oxy)methyl)phenyl)butyl)acetamide.

The specific synthetic process and conditions for the compound are as follows:

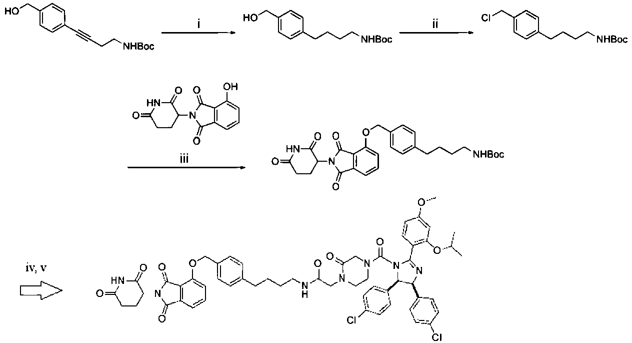

Reagents and conditions: (i) H₂, Pd/C, ethyl acetate, rt, 2 h; (ii) MsCl, NEt₃, CH₂Cl₂, 0°C->rt, 20 h; (iii) K₂CO₃, DMF, 60°C, 4 h; (iv) TFA, CH₂Cl₂, rt, 1 h; (v) nutlin acid, HATU, DIPEA, DMF, rt, 16 h.

In a specific embodiment of the present invention, the compound may be:
2-(4-(*cis*-4,5-bis(4-chlorophenyl)-2-(2-isopropoxy-4-methoxyphenyl)-4,5-dihydro-1H-imidazole-1-carbonyl)-2-oxopiperazin-1-yl)-N-(9-(2-(2,6-dioxopiperidin-3-yl)-1,3-dioxoisoindolin-4-yl)nonyl)acetamide.

The specific synthetic procedures and conditions for the compounds are as follows:

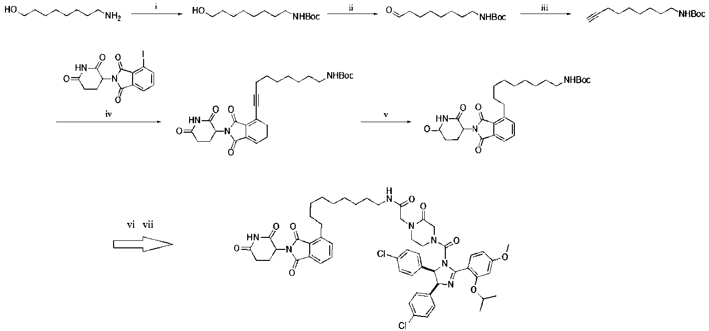

Reagents and conditions: (i) (Boc)₂O, CH₂Cl₂, 0°C->rt, 3 h; (ii) PCC, Al₂O₃, CH₂Cl₂, rt, 3 h; (iii) Dimethyl (1-diazo-2-oxopropyl)phosphonate, K₂CO₃, CH₃OH, rt, 4 h; (iv) CuI, PdCl₂(PPh₃)₂, Et3N, DMF, 80°C; (v) H₂, Pd/C, ethanol, rt, 63 h; (vi) TFA, CH₂Cl₂, rt, 1 h; (vii) Nutlin acid, HATU, DIPEA, DMF, rt, 16 h.

In a specific embodiment of the present invention, the compound may be:
2-(4-(*cis*-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-((4-(((2-(2,6-dioxopiperidin-3-yl)-1,3-dioxoisoindolin-4-yl)oxy)methyl)benzyl)oxy)ethyl)acetamide.

The specific synthetic process and conditions for the compound are as follows:

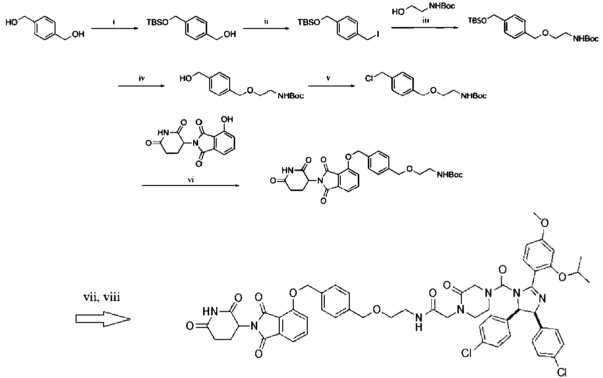

Reagents and conditions: (i) imidazole, TBSCl, THF, 0°C to rt, 12 h; (ii) PPh₃, imidazole, I₂, Et ₂O/MeCN (3:1), rt, 1 h; (iii) TBAI, KOH, THF, rt, 40 h; (iv) TBAF, THF, rt, 3 h; (v) MsCl, DIPEA, DCM, 0°C->rt, 16 h; (vi) NaHCO₃, KI, DMF, 80°C, 16 h; (vii) TFA, CH₂Cl₂, rt, 1 h; (viii) nutlinic acid, HATU, DIPEA, DMF, rt, 16 h.

Compounds may contain asymmetric centers and thus may include enantiomers and diastereomers. The term "enantiomers" refer to two stereoisomers of a compound that are non-superimposable mirror images of each other. When compounds disclosed herein have two or more asymmetric centers, they may be also present as diastereomers. Enantiomers and diastereomers belong to the broader category of stereoisomers. All possible stereoisomers are intended to include substantially pure isolated enantiomers, the racemic mixtures thereof, and mixtures of diastereomers. The stereoisomers are intended to include all stereoisomers of the compounds disclosed herein and/or pharmaceutically acceptable salts thereof. Unless otherwise stated, reference to one isomer applies to all possible isomers. Whenever an isomer composition is not specified, all possible isomers are included.

The term "substantially pure" means that the target stereoisomer is present in an amount of 35% or less, for example, 30% or less, further 25% or less, or even 20% or less, based on the weight of the other stereoisomer(s). In some embodiments, the term "substantially pure" means that the target stereoisomer is present in an amount of 10% or less, for example, 5% or less, even 1% or less, based on the weight of the other stereoisomer.

The term "pharmaceutically acceptable salt" may include an acid addition salt formed with pharmaceutically acceptable free acids, and the free acid may be an organic or inorganic acid.

Examples of the organic acid include, but are not limited to, citric acid, acetic acid, lactic acid, tartaric acid, maleic acid, fumaric acid, formic acid, propionic acid, oxalic acid, trifluoroacetic acid, benzoic acid, gluconic acid, metasulfonic acid, glycolic acid, succinic acid, 4-toluenesulfonic acid, glutamic acid, and aspartic acid. Examples of the inorganic acid include, but are not limited to, hydrochloric acid, bromic acid, sulfuric acid, and phosphoric acid.

For example, when the compound is an anion or has a functional group that may be an anion (e.g., -COOH may be -COO-), a salt may be formed with an appropriate cation. Examples of suitable inorganic cations include, but are not limited to, alkali metal ions such as Na⁺ and K⁺, alkaline earth metal cations such as Ca²⁺ and Mg²⁺, and other cations such as Al³⁺. Examples of suitable organic cations include, but are not limited to, ammonium ions (i.e., NH⁴⁺) and substituted ammonium ions (e.g., NH₃R⁺, NH₂R₂⁺, NHR₃⁺, NR₄⁺).

Examples of suitable substituted ammonium ions include those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids such as lysine and arginine. A common example of a quaternary ammonium ion is N(CH₃)⁴.

When a compound is a cation or has a functional group that may be a cation (e.g., -NH₂ may be -NH₃⁺), it may form a salt with a suitable anion. Examples of suitable inorganic anions include, but are not limited to, those derived from the following inorganic acids: hydrochloric acid, hydrobromic acid, hydroiodic acid, sulfuric acid, sulfurous acid, nitric acid, nitrous acid, phosphoric acid, and phosphorous acid.

Examples of suitable organic anions include, but are not limited to, those derived from the following organic acids: 2-acetyoxybenzoic acid, acetic acid, ascorbic acid, aspartic acid, benzoic acid, camphorsulfonic acid, cinnamic acid, citric acid, edetic acid, ethanedisulfonic acid, ethanesulfonic acid, fumaric acid, glutaptonic acid, gluconic acid, glutamic acid, glycolic acid, hydroxymaleic acid, hydroxynaphthalenecarboxylic acid, isethionic acid, lactic acid, lactobionic acid, lauric acid, maleic acid, malic acid, methanesulfonic acid, mucic acid, oleic acid, oxalic acid, palmitic acid, pamoic acid, pantothenic acid, phenylacetic acid, phenylsulfonic acid, propionic acid, pyruvic acid, salicylic acid, stearic acid, succinic acid, sulfanilic acid, tartaric acid, toluenesulfonic acid, and valeric acid. Examples of suitable polymeric organic anions include, but are not limited to, those derived from the following polymeric acids: tannic acid, carboxymethyl cellulose, and the like.

In another aspect, the present invention is directed to a composition for inducing osteogenic differentiation of stem cells containing the compound, the stereoisomer thereof, or the pharmaceutically acceptable salt thereof.

The osteogenic differentiation may be the differentiation of osteocytes or stem cells into osteoblasts.

The term "stem cell" refers to pluripotent or totipotent self-renewing cells that are capable of differentiating into cells of all tissues of an organism. They include embryonic stem cells, induced pluripotent stem cells, and adult stem cells.

The term "embryonic stem cells" refers to embryonic stem cells derived from the fertilized egg created by combination of sperm, a male gamete, with an egg, a female gamete. Embryonic stem cells are obtained from the inner cell mass of a preimplantation blastocyst embryo. They have the ability to differentiate into cells of all tissues, yet are undifferentiated and can be indefinitely proliferated under suitable culture conditions. In other words, in a specialized differentiation culture environment, cell differentiation is induced in a desired direction and used as cell therapies for diseases such as neurological diseases, diabetes, and blood diseases. Therefore, numerous patients are expected to be treated with only a single embryonic stem cell line.

The term "induced pluripotent stem cells (iPSCs)" refers to differentiated cells that have been artificially reprogrammed to have pluripotent differentiation potential.

The term "adult stem cells" refers to primitive cells isolated from mammalian tissues, including humans, just before differentiation. They have the ability to proliferate indefinitely and differentiate into various cell types (e.g., adipocytes, chondrocytes, muscle cells, bone cells, or the like).

The term "differentiation" refers to the phenomenon in which less specialized cells develop into specific cells with changed size, shape, membrane potential, metabolic activity, and response to signals. This differentiation primarily occurs during the process in which multicellular organisms form complicated tissues from a single zygote, or adult stem cells are differentiated into specific cells during tissue repair in adults.

The composition according to the present invention may be a pharmaceutical composition.

The pharmaceutical composition according to the present invention may be administered via any route. The composition of the present invention may be administered to an animal directly (e.g., topically, by injection, transplantation, or local administration to a tissue site) or systemically (e.g., parenterally or orally) by any suitable means. When the composition of the present invention is administered parenterally, such as intravenously, subcutaneously, ophthalmically, intraperitoneally, intramuscularly, orally, rectally, intraorbitally, intracerebrally, intracranially, intraspinally, intraventricularly, intrathecally, intracisternally, intracapsularly, intranasally, or as an aerosol, the pharmaceutical composition may contain, for example, portions of aqueous or physiologically acceptable fluid suspensions or solutions. Accordingly, a physiologically acceptable carrier or vehicle may be added to the composition and delivered to the patient. Thus, a carrier, such as a fluid for the preparation, typically includes a saline solution.

Furthermore, the frequency of administration varies depending on the pharmacokinetic parameters of the formulation used. Typically, clinicians will administer the pharmaceutical composition until the desired effect is achieved. Thus, the pharmaceutical composition may be administered as a single dose, as two or more doses at predetermined intervals, or as a continuous infusion via an implantable device or catheter. Further refinement of the appropriate dosage is routinely accomplished by those skilled in the art and falls within the scope of routine practice.

Moreover, the unit dosage for humans is typically 0.01 µg/kg to 100 mg/kg, specifically 1 µg/kg to 10 mg/kg of body weight. This dosage is optimal, but it may vary depending on the disease in need of treatment and the presence or absence of side effects. The optimal dosage may be determined through routine experimentation. Administration of the fusion protein may be carried out by periodic bolus injections, or continuous intravenous, subcutaneous, or intraperitoneal administration from an external reservoir (e.g., an intravenous bag) or internal source (e.g., a bioerodible implant).

Suitable routes include oral, parenteral (including subcutaneous, intramuscular, intravenous, intraarterial, inhalation, intradermal, intrathecal or transdural administration, and infusion), transdermal, rectal, intranasal, topical (including buccal and sublingual), vaginal, intraperitoneal, intrapulmonary, and intranasal administration. Topical administration may include transdermal delivery, such as through a transdermal patch or iontophoresis device.

Preferred routes may vary depending on, for example, the condition of the recipient. For oral administration, the composition may be formulated into a pill, capsule, tablet, or the like along with a pharmaceutically acceptable carrier, lubricant or excipient. For parenteral administration, the composition may be formulated into a unit dose injection along with a pharmaceutically acceptable parenteral vehicle or diluent.

The pharmaceutical composition may further contain a pharmaceutically acceptable carrier and the pharmaceutically acceptable carrier may include, but is not limited to, at least one selected from lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil that are commonly used for formulation of drugs. The pharmaceutical composition may further contain at least one selected from the group consisting of a diluent, an excipient, a lubricant, a wetting agent, a sweetener, a flavoring agent, an emulsifier, a suspending agent, and a preservative that are commonly used in the preparation of pharmaceutical compositions.

The pharmaceutical composition may be orally or parenterally administered in an effective amount. The parenteral administration includes intravenous injection, subcutaneous injection, intramuscular injection, intraperitoneal injection, intradermal administration, topical administration, intranasal administration, intrapulmonary administration, intrarectal administration or the like. Upon oral administration, since proteins or peptides are digested, an oral composition should be coated with an active drug or formulated so as to protect the same from degradation in the stomach. In addition, the composition may be administered using any device capable of delivering the active substance to target cells.

As used herein, the term "treatment" refers to any indication of success in any subjective or objective parameter, for example, relief; remission; production of symptoms or damage more tolerable to the patient and reduction in pathology or condition; slowing of degeneration or deterioration; achieving a terminal degenerative state that is less debilitating; and the treatment or amelioration of an injury, pathology or condition including improvement of the patient's physical or mental well-being. Treatment or alleviation of symptoms may be based on objective or subjective parameters including physical examination, neuropsychiatric examination and/or psychological medical evaluation.

The term "effective amount" is generally an amount that is sufficient to reduce the severity or frequency of symptoms, eliminate the symptoms and underlying causes, prevent the occurrence or underlying causes of symptoms, or ameliorate or correct damage resulting from or related to a disease condition.

An effective amount is a therapeutically effective amount or a prophylactically effective amount. The "therapeutically effective amount" is an amount that is sufficient to correct disease conditions or symptoms, in particular, conditions or symptoms associated with disease conditions, or otherwise prevent, hinder, delay or reverse the progression of disease conditions or any other undesirable symptoms associated in any way with diseases. The prophylactically effective amount refers to an amount of a pharmaceutical composition that has an intended prophylactic effect, e.g., an effect of preventing or delaying the onset of a disease condition, or reducing the likelihood of onset (or recurrence) of disease conditions or related symptoms when administered to subjects.

### [Example]

Hereinafter, the present invention will be described in more detail with reference to examples. However, it will be obvious to those skilled in the art that these examples are provided only for illustration of the present invention and should not be construed as limiting the scope of the present invention.

### Preparation Example 1. Materials and Methods

Unless otherwise specified, reagents were commercially available and used without further purification. Anhydrous THF, DMF, DCM, ACN, and DMSO were purchased from Sigma-Aldrich. Pyridinium chlorochromate (PCC), 1-[bis(dimethylamino)methylene]-1H-1,2,3-triazolo[4,5-b]pyridinium 3-oxide hexafluophosphate (HATU), dimethyl (1-diazo-2-oxopropyl)phosphonate, copper(I) iodide (CuI), and bis(triphenylphosphine)palladium(II) dichloride (PdCl₂(PPh₃)₂), (Boc)₂O (di-tert-butyl dicarbonate), DIPEA (N,N-diisopropylethylamine), and TFA (trifluoroacetic acid) were purchased from Sigma-Aldrich, Acros Organics, Alfa Aesar, Angele, Combi-blocks, or TCI were purchased and used without any treatment unless otherwise stated. Unless otherwise stated, reactions were performed under a N₂ atmosphere using anhydrous solvents. Unless otherwise stated, the reaction temperature was controlled by an IKAmag thermostat. Analytical thin layer chromatography (TLC) was performed using TLC silica gel 60 F254 (Merck) and visualized by UV fluorescence quenching, ninhydrin, and KMnO₄ staining. Flash column chromatography was performed on a Biotage^{®} Selekt Flash Purification System using Biotage^{®} Sfar Silica and Biotage^{®} Sfar C18 D-Duo 100Å cartridges. Preparative HPLC was performed on an Agilent 1260 system with an Agilent 10 prepC18 column (100Å, length 250 mm, i.d. 21.2 mm, 10 µm) at a solvent flow rate of 20 mL/min. All NMR spectra were recorded on a Bruker JNM-ECZR 600 MHz spectrometer. ¹H NMR spectra were reported relative to residual CDCl₃ (δ 7.26 ppm), CD₃OD (3.31 ppm), or (CD₃)₂SO₂ (δ 2.50 ppm). ¹³C NMR spectra were reported relative to residual CD₃OD (δ 49.00 ppm). ¹H and ¹³C data were reported in chemical shifts (δ ppm). IR spectra were recorded on a Nicolet iS50 FT-IR spectrometer using the ATR method and reported in wavenumber (cm⁻¹). HRMS was obtained on a Bruker ultra high-resolution ESI Q-TOF mass spectrometer.

### Preparation Example 2. Method

### Preparation Example 2-1. Synthesis of 6a-f:

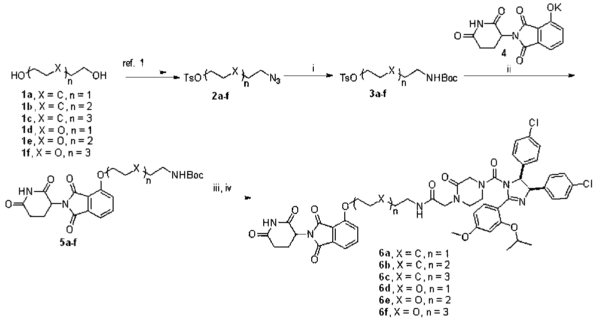

Reagents and conditions: (i) H₂, Pd/C, (Boc)₂O, THF, rt, 1 h; (ii) DIPEA, CH₃CN, 70°C, 16 h; (iii) TFA, CH₂Cl₂, rt, 1 h; (iv) nutlinic acid, HATU, DIPEA, DMF, rt, 16 h.

### A. General method for synthesizing 3a to f:

(Boc)₂O (1.20 equiv) was added to a stirred solution of azide (1.00 equiv) in anhydrous THF. The reaction mixture was purged with nitrogen, and 10% Pd/C (0.10 equiv) was then added thereto. The reaction mixture was stirred under a hydrogen atmosphere (balloon pressure) at 25°C for 1 hour. After completion of the reaction, the mixture was filtered through Celite and washed with ethyl acetate. The filtrate was concentrated under reduced pressure. The crude product was eluted with ethyl acetate/hexane and purified by flash column chromatography on silica gel, to obtain the desired product with a yield of 68 to 80%.

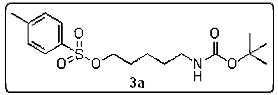

5-((tert-butoxycarbonyl)amino)pentyl 4-methylbenzenesulfonate, 3a: The title compound was synthesized using General Method A and obtained as a yellow liquid in 68% yield.

¹H NMR (600 MHz, CDCl₃) δ 7.80-7.77 (m, 2H), 7.34 (d, J = 8.0 Hz, 2H), 4.49 (bs, 1H), 4.01 (t, J = 6.4 Hz, 2H), 3.10-3.01 (m, 2H), 2.45 (s, 3H), 1.68-1.62 (m, 2H), 1.46-1.39 (m, 11H), 1.36-1.30 (m, 2H).

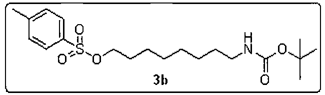

8-((tert-butoxycarbonyl)amino)octyl 4-methylbenzenesulfonate, 3b: The title compound was synthesized using General Method A and obtained as a colorless viscous liquid with a yield of 74%.

¹H NMR (600 MHz, CDCl₃) δ 7.80-7.77 (m, 2H), 7.34 (d, J = 8.0 Hz, 2H), 4.49 (bs, 1H), 4.01 (t, J = 6.5 Hz, 2H), 3.08 (q, J = 6.7 Hz, 2H), 2.45 (s, 3H), 1.66-1.56 (m, 4H), 1.44 (s, 9H), 1.33-1.18 (m, 8H).

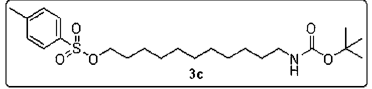

11-((tert-butoxycarbonyl)amino)undecyl 4-methylbenzenesulfonate, 3c: The title compound was synthesized using General Method A and obtained as a white, sticky solid with a yield of 80%.

¹H NMR (600 MHz, CDCl₃) δ 7.80-7.77 (m, 2H), 7.34 (d, J = 8.0 Hz, 2H), 4.49 (bs, 1H), 4.01 (t, J = 6.5 Hz, 2H), 3.12-3.04 (m, 2H), 2.45 (s, 3H), 1.67-1.59 (m, 3H), 1.44 (s, 9H), 1.32-1.17 (m, 15H).

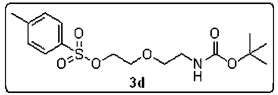

2-(2-((tert-butoxycarbonyl)amino)ethoxy)ethyl 4-methylbenzenesulfonate, 3d: The title compound was synthesized using General Method A and obtained as a colorless rubbery liquid with a yield of 70%.

¹H NMR (600 MHz, CDCl₃) δ 7.82-7.79 (m, 2H), 7.35 (d, J = 8.0 Hz, 2H), 4.80 (bs, 1H), 4.19-4.14 (m, 2H), 3.64-3.61 (m, 2H), 3.45 (t, J = 5.2 Hz, 2H), 3.26-3.20 (m, 2H), 2.45 (s, 3H), 1.45 (s, 9H).

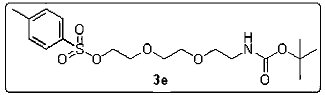

2,2-Dimethyl-4-oxo-3,8,11-trioxa-5-azatridecan-13-yl 4-methylbenzenesulfonate 6, 3e: The title compound was synthesized using General Method A and was obtained as a pale yellow, rubbery liquid.

¹H NMR (400 MHz, CDCl₃) δ 7.80 (d, J = 8.0 Hz, 2H), 7.34 (d, J = 8.0 Hz, 2H), 5.30 (s, 0H), 4.94 (s, 1H), 4.17 (t, J = 4.8 Hz, 2H), 3.69 (t, J = 4.9 Hz, 2H), 3.56 (qd, J = 4.9, 1.9 Hz, 4H), 3.50 (t, J = 5.2 Hz, 2H), 3.29 (q, J = 5.5 Hz, 2H), 2.45 (s, 3H), 1.67-1.56 (m, 0H), 1.43 (s, 9H).

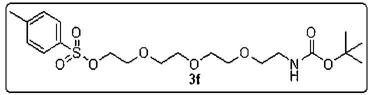

2,2-Dimethyl-4-oxo-3,8,11,14-tetraoxa-5-azahexadecan-16-yl-4-methylbenzenesulfonate, 3f: The title compound was synthesized using general method A and obtained as a colorless rubbery liquid in 77% yield.

¹H NMR (600 MHz, CDCl₃) δ 7.81-7.78 (m, 2H), 7.34 (d, J = 7.9 Hz, 2H), 5.02 (bs, 1H), 4.18-4.15 (m, 2H), 3.71-3.68 (m, 2H), 3.62-3.57 (m, 8H), 3.52 (t, J = 5.2 Hz, 2H), 3.32-3.26 (m, 2H), 2.44 (s, 3H), 1.43 (s, 9H).

B. General method for the synthesis of 5a-f: potassium 2-(2,6-dioxopiperidin-3-yl)-1,3-dioxoisoindolin-4-oleate (1.00 equiv) and DIPEA (10.00 equiv) were added to a stirred solution of tosyl (1.10 equiv) in anhydrous acetonitrile. The reaction mixture was stirred in a sealed tube at 70°C for 16 hours. After completion of the reaction, the mixture was cooled to room temperature, filtered through Celite, and washed with ethyl acetate. The filtrate was removed under reduced pressure and the crude product was purified on silica gel by flash column chromatography, eluting with acetone/hexane, to obtain the desired product with a yield of 40 to 85%.

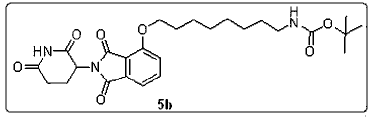

Tert-butyl (5-((2-(2,6-dioxopiperidin-3-yl)-1,3-dioxoisoindolin-4-yl)oxy)pentyl)carbamate, 5a: The title compound was obtained as an off-white solid with a yield of 55% using Method B.

¹H NMR (600 MHz, CDCl₃) δ 7.96 (s, 1H), 7.67 (dd, J = 8.4, 7.3 Hz, 1H), 7.46 (d, J = 7.1 Hz, 1H), 7.21 (d, J = 8.4 Hz, 1H), 4.95 (dd, J = 12.6, 5.4 Hz, 1H), 4.62 (s, 1H), 4.22-4.14 (m, 2H), 3.18-3.11 (m, 2H), 2.93-2.87 (m, 1H), 2.85-2.78 (m, 1H), 2.73 (ddd, J = 16.9, 13.6, 5.0 Hz, 1H), 2.16-2.11 (m, 1H), 1.93-1.88 (m, 2H), 1.61-1.52 (m, 4H), 1.44 (s, 9H).

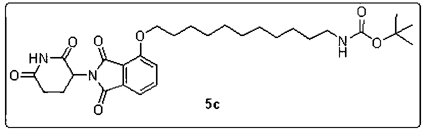

tert-Butyl (8-((2-(2,6-dioxopiperidin-3-yl)-1,3-dioxoisoindolin-4-yl)oxy)octyl)carbamate, 5b: The title compound was obtained as a yellow solid with a yield of 50% using method B.

¹H NMR (600 MHz, CDCl₃) δ 8.12 (s, 1H), 7.67 (dd, J = 8.5, 7.3 Hz, 1H), 7.45 (d, J = 7.1 Hz, 1H), 7.21 (d, J = 8.5 Hz, 1H), 4.99-4.92 (m, 1H), 4.54 (s, 1H), 4.21-4.13 (m, 2H), 3.14-3.08 (m, 2H), 2.92-2.87 (m, 1H), 2.86-2.79 (m, 1H), 2.77-2.70 (m, 1H), 2.16-2.09 (m, 1H), 1.87 (p, J = 7.4, 6.9 Hz, 2H), 1.53-1.42 (m, 13H), 1.40-1.29 (m, 6H).

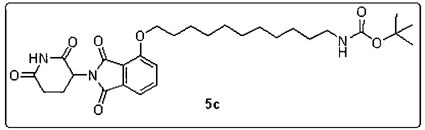

tert-Butyl (11-((2-(2,6-dioxopiperidin-3-yl)-1,3-dioxoisoindolin-4-yl)oxy)undecyl)carbamate 5c: The title compound was obtained as an off-white solid with a yield of 40% using Method B.

¹H NMR (600 MHz, CDCl₃) δ 7.98 (s, 1H), 7.66 (dd, J = 8.5, 7.3 Hz, 1H), 7.44 (d, J = 7.3 Hz, 1H), 7.20 (d, J = 8.5 Hz, 1H), 4.94 (dd, J = 12.5, 5.4 Hz, 1H), 4.49 (bs, 1H), 4.16 (t, J = 6.6 Hz, 2H), 3.12-3.05 (m, 2H), 2.91-2.85 (m, 1H), 2.81 (td, J = 12.9, 4.1 Hz, 1H), 2.72 (ddd, J = 16.7, 13.5, 5.1 Hz, 1H), 2.14-2.09 (m, 1H), 1.87 (dt, J = 14.4, 6.7 Hz, 2H), 1.51-1.41 (m, 13H), 1.37-1.23 (m, 12H).

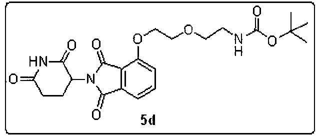

tert-Butyl(2-(2-((2-(2,6-dioxopiperidin-3-yl)-1,3-dioxoisoindolin-4-yl)oxy)ethoxy-y)ethyl)carbamate, 5d: The title compound was synthesized using General Method B and obtained as an off-white solid with a yield of 86%.

¹H NMR (600 MHz, CDCl₃) δ 7.98 (s, 1H), 7.69 (dd, J = 8.4, 7.3 Hz, 1H), 7.49 (dd, J = 7.3, 0.7 Hz, 1H), 7.27-7.25 (m, 1H), 5.04 (bs, 1H), 4.96 (dd, J = 12.6, 5.4 Hz, 1H), 4.34 (t, J = 4.8 Hz, 2H), 3.92-3.89 (m, 2H), 3.66 (t, J = 5.2 Hz, 2H), 3.37-3.30 (m, 2H), 2.93-2.87 (m, 1H), 2.83 (qd, J = 12.7, 4.0 Hz, 1H), 2.73 (ddd, J = 16.9, 13.6, 5.0 Hz, 1H), 2.16-2.10 (m, 1H), 1.42 (s, 9H).

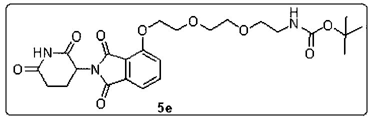

tert-Butyl (2-(2-(2-((2-(2,6-dioxopiperidin-3-yl)-1,3-dioxoisoindolin-4-yl)oxy)ethoxy)ethoxy)ethyl)carbamate, 5e: The title compound was synthesized using General Method B and obtained as an off-white solid with a yield of 64%.

¹H NMR (600 MHz, CDCl₃) δ 8.20 (s, 1H), 7.68 (dd, J = 8.3, 7.4 Hz, 1H), 7.48 (d, J = 7.3 Hz, 1H), 7.27-7.25 (m, 1H, mixed with CDCl₃), 5.03 (s, 1H), 4.95 (dd, J = 12.4, 4.4 Hz, 1H), 4.37-4.34 (m, 2H), 3.96-3.93 (m, 2H), 3.78 (t, J = 4.5 Hz, 2H), 3.63 (t, J = 4.6 Hz, 2H), 3.57-3.52 (m, 2H), 3.33-3.27 (m, 2H), 2.91-2.86 (m, 1H), 2.81 (ddd, J = 24.5, 12.4, 3.5 Hz, 1H), 2.77-2.69 (m, 1H), 2.15-2.10 (m, 1H), 1.43 (s, 9H).

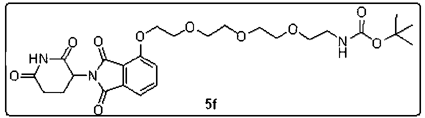

tert-Butyl (2-(2-(2-(2-((2-(2,6-dioxopiperidin-3-yl)-1,3-dioxoisoindolin-4-yl)-oxy)-ethoxy)ethoxy)ethoxy)ethyl)carbamate, 5f: The title compound was synthesized using the general method B, and was obtained as an off-white solid with a yield of 68%.

¹H NMR (600 MHz, CDCl₃) δ 8.19 (s, 1H), 7.67 (dd, J = 8.5, 7.3 Hz, 1H), 7.47 (d, J = 7.1 Hz, 1H), 7.27 (d, J = 8.5 Hz, 1H), 5.06 (s, 1H), 4.95 (dd, J = 12.5, 5.4 Hz, 1H), 4.36 (t, J = 4.8 Hz, 2H), 3.96-3.94 (m, 2H), 3.81-3.78 (m, 2H), 3.68-3.65 (m, 2H), 3.65-3.58 (m, 4H), 3.53 (t, J = 5.2 Hz, 2H), 3.33-3.27 (m, 2H), 2.92-2.86 (m, 1H), 2.86-2.77 (m, 1H), 2.73 (ddd, J = 16.8, 13.6, 4.9 Hz, 1H), 2.15-2.09 (m, 1H), 1.43 (s, 9H).

C. General method for NH-Boc deprotection: trifluoroacetic acid (10.00 equiv) was added dropwise to a stirred solution of Boc-amine (1.00 equiv) in anhydrous CH₂Cl₂ at 0°C. The reaction mixture was stirred at 25°C for 1 hour. After completion of the reaction, the mixture was concentrated under vacuum and the residue was co-evaporated with toluene (twice), to obtain the desired product as a TFA salt. The crude product was used in the next step without further purification.

D. General method for the synthesis of 6a-f: HATU (1.50 equiv) and DIPEA (5.00 equiv) were added to a stirred solution of nutrylic acid (1.20 equiv) in anhydrous DMF at 25°C. The reaction mixture was stirred at the same temperature for 30 minutes, and a solution of the TFA salt or free amine (1.00 equivalents) in anhydrous DMF was added dropwise thereto. The reaction mixture was stirred at 25°C for 16 hours. After completion of the reaction, the result was diluted with water and ethyl acetate, and stirred vigorously. The organic layer was separated and the aqueous layer was extracted with ethyl acetate. The combined organic layers were washed with saturated aqueous LiCl solution and brine, dried over anhydrous sodium sulfate, filtered, and concentrated under vacuum.

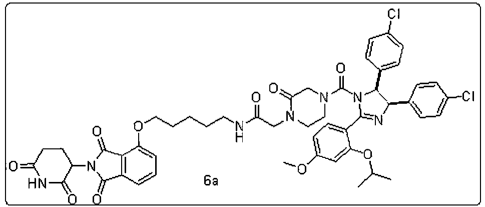

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(5-((2-(2,6-dioxopiperidin-3-yl)-1,3-dioxoisoindolin-4-yl)oxy)pentyl)acetamide, 6a: The title compound was synthesized using General Method C and then General Method D. The crude product was purified by silica gel column chromatography on a Biotage system, eluting with methanol/DCM. The result was secondarily purified by prep-HPLC and the product was obtained as an off-white solid with a yield of 28%.

¹H NMR (600 MHz, CD₃OD) δ 7.75 (dd, J = 8.5, 7.3 Hz, 1H), 7.57 (dd, J = 8.3, 3.1 Hz, 1H), 7.42 (t, J = 7.8 Hz, 2H), 7.13 (d, J= 8.4 Hz, 2H), 7.07 (d, J = 8.2 Hz, 2H), 7.02 (dd, J = 8.4, 1.5 Hz, 2H), 6.94 (d, J = 8.0 Hz, 2H), 6.66 (m, 2H), 5.74 (d, J = 10.1 Hz, 1H), 5.58-5.53 (m, 1H), 5.09 (dd, J = 12.5, 5.5 Hz, 1H), 4.73 (p, J = 6.0 Hz, 1H), 4.62 (s, 3H), 4.21 (t, J = 6.0 Hz, 2H), 4.00-3.88 (m, 1H), 3.87 (s, 3H), 3.86-3.73 (m, 3H), 3.52 (m, 1H), 3.41 (m, 1H), 3.24-3.19 (m, 1H), 3.07-3.01 (m, 2H), 2.89-2.78 (m, 1H), 2.75-2.65 (m, 2H), 2.08 (m, 1H), 1.86 (q, J = 6.7 Hz, 2H), 1.57 (m, 5H), 1.38-1.32 (m, 6H).

¹³C NMR (151 MHz, CD₃OD) δ 174.7, 174.6, 171.6, 171.6, 169.7, 168.7, 167.5, 167.3, 165.2, 163.4, 158.6, 158.0, 155.7, 138.0, 137.4, 136.3, 135.1, 134.3, 133.2, 130.7, 130.0, 129.1, 129.0, 120.5, 118.1, 116.4, 113.4, 106.5, 101.2, 72.4, 71.8, 70.3, 70.1, 56.2, 50.6, 50.4, 50.3, 48.1, 43.2, 40.1, 40.1, 32.2, 29.8, 29.5, 24.2, 23.7, 22.5, 22.4.

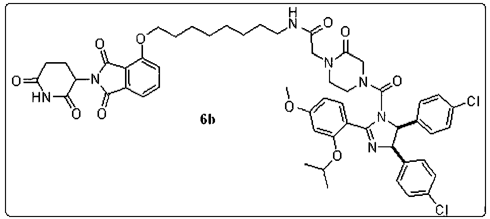

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(8-((2-(2,6-dioxoopiperidin-3-yl)-1,3-dioxoisoindolin-4-yl)oxy)octyl)acetamide, 6b: The title compound was synthesized using General Method C and then General Method D. The crude product was purified by silica gel column chromatography on a Biotage system, eluting with methanol/DCM. The result was secondarily purified by preparative HPLC and the product was obtained as an off-white solid with a yield of 24%.

¹H NMR (600 MHz, CD₃OD) δ 7.78-7.74 (m, 1H), 7.58 (dd, J = 8.4, 1.3 Hz, 1H), 7.45-7.41 (m, 2H), 7.15-7.12 (m, 2H), 7.08 (d, J = 7.7 Hz, 2H), 7.03 (d, J = 8.3 Hz, 2H), 6.94 (d, J = 8.0 Hz, 2H), 6.67 (dd, J = 8.4, 2.2 Hz, 1H), 6.65 (d, J = 2.3 Hz, 1H), 5.75 (d, J = 10.1 Hz, 1H), 5.56 (d, J = 10.1 Hz, 1H), 5.09 (dd, J = 12.7, 5.5 Hz, 1H), 4.73 (p, J = 6.1 Hz, 1H), 4.22 (t, J = 6.3 Hz, 2H), 3.91 (dd, J = 16.2, 3.5 Hz, 1H), 3.88 (s, 3H), 3.85-3.76 (m, 3H), 3.56-3.49 (m, 1H), 3.43-3.37 (m, 1H), 3.16 (t, J = 7.0 Hz, 2H), 3.10-2.99 (m, 2H), 2.86 (ddd, J = 19.0, 13.9, 5.3 Hz, 1H), 2.78-2.66 (m, 2H), 2.10 (dd, J = 10.1, 4.7 Hz, 1H), 1.84 (dt, J = 14.0, 6.4 Hz, 2H), 1.50 (dq, J = 28.7, 7.3 Hz, 4H), 1.41-1.31 (m, 12H).

¹³C NMR (151 MHz, CD₃OD) δ 174.6, 171.6, 169.6, 168.7, 167.4, 165.0, 163.2, 158.6, 158.1, 155.8, 138.0, 137.6, 136.5, 135.2, 134.2, 133.1, 130.7, 130.0, 129.1, 129.0, 120.6, 118.1, 116.3, 113.8, 106.5, 101.2, 72.4, 72.1, 70.5, 70.1, 56.2, 50.6, 50.4, 50.3, 48.2, 43.2, 40.4, 32.2, 30.3, 30.1, 30.0, 27.7, 26.8, 23.7, 22.5, 22.4.

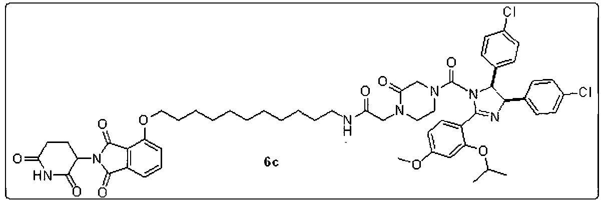

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(11-((2-(2,6-dioxoperydin-3-yl)-1,3-dioxoisoindolin-4-yl)oxy)undecyl)acetamide, 6c: The title compound was synthesized using General Method C followed by General Method D. The crude product was purified by C-18 silica gel column chromatography on a Biotage system, eluting with acetonitrile/deionized water. The result was secondarily purified by preparative HPLC and the product was obtained as an off-white solid with a yield of 48%.

¹H NMR (600 MHz, CD₃OD) δ 7.77-7.73 (m, 1H), 7.59 (d, J = 8.3 Hz, 1H), 7.44-7.40 (m, 2H), 7.13 (d, J = 8.5 Hz, 2H), 7.08 (d, J = 8.8 Hz, 2H), 7.03 (d, J = 8.5 Hz, 2H), 6.95 (d, J = 8.1 Hz, 2H), 6.67 (dd, J = 8.4, 2.3 Hz, 1H), 6.65 (d, J = 2.2 Hz, 1H), 5.75 (d, J = 10.1 Hz, 1H), 5.56 (d, J = 10.1 Hz, 1H), 5.09 (dd, J = 12.4, 5.5 Hz, 1H), 4.73 (p, J = 6.0 Hz, 1H), 4.22-4.19 (m, 2H), 3.91 (d, J = 16.1 Hz, 1H), 3.88 (s, 3H), 3.85-3.76 (m, 3H), 3.53 (dt, J = 13.9, 5.2 Hz, 1H), 3.44-3.36 (m, 1H), 3.14 (t, J = 7.1 Hz, 2H), 3.10-3.00 (m, 2H), 2.86 (ddd, J = 18.3, 14.2, 5.4 Hz, 1H), 2.77-2.66 (m, 2H), 2.13-2.07 (m, 1H), 1.88-1.81 (m, 2H), 1.56-1.43 (m, 4H), 1.42-1.25 (m, 18H).

¹³C NMR (151 MHz, CD₃OD) δ 174.6, 171.5, 169.6, 168.7, 167.3, 165.0, 163.2, 158.5, 158.1, 155.83, 137.93, 137.58, 136.47, 135.13, 134.19, 133.09, 130.72, 129.96, 129.08, 129.00, 120.52, 118.12, 116.28, 113.82, 106.43, 101.23, 72.42, 72.07, 70.51, 70.07, 56.16, 50.54, 50.40, 50.23, 48.17, 43.16, 40.45, 32.20, 30.57, 30.49, 30.36, 30.33, 30.31, 29.99, 27.89, 26.91, 23.68, 22.49, 22.44.

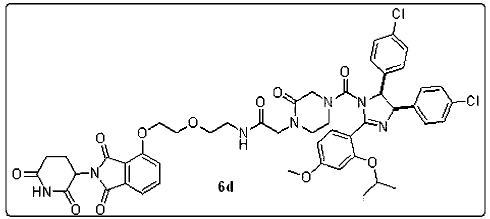

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-((2-(2,6-dioxopiperidin-3-yl)-1,3-dioxoisoindolin-4-yl))oxy)ethoxy)ethyl)acetamide, 6d: The title compound was synthesized using General Method C and then General Method D. The crude product was purified by amine column chromatography on a Biotage system, eluting with methanol/DCM. The result was secondarily purified by preparative HPLC and the product was obtained as an off-white solid with a yield of 28%.

¹H NMR (600 MHz, CD₃OD) δ 7.79 (dd, J = 8.5, 7.3 Hz, 1H), 7.59 (dd, J = 8.3, 2.2 Hz, 1H), 7.49-7.44 (m, 2H), 7.16 (d, J = 8.5 Hz, 2H), 7.12-7.09 (m, 2H), 7.04 (d, J = 8.4 Hz, 2H), 6.96 (d, J = 8.0 Hz, 2H), 6.71-6.67 (m, 2H), 5.82 (d, J = 10.0 Hz, 1H), 5.64 (t, J = 9.2 Hz, 1H), 5.10 (dd, J = 12.5, 5.3 Hz, 1H), 4.77 (td, J = 5.9, 2.3 Hz, 1H), 4.34 (t, J = 4.0 Hz, 2H), 3.94-3.81 (m, 5H), 3.83-3.73 (m, 2H), 3.65 (t, J = 5.2 Hz, 2H), 3.41 (m, 3H), 3.05 (m, 2H), 2.89-2.80 (m, 1H), 2.76-2.66 (m, 2H), 2.09 (m, 1H), 1.39 (dd, J = 6.0, 1.1 Hz, 3H), 1.37-1.28 (m, 6H).

¹³C NMR (151 MHz, CD₃OD) δ 174.6, 174.6, 171.5, 169.9, 168.5, 167.7, 167.4, 165.1, 163.2, 158.6, 157.8, 155.9, 138.1, 137.6, 136.6, 136.5, 135.1, 134.2, 133.2, 130.8, 130.0, 129.1, 129.0, 120.8, 118.3, 116.8, 106.4, 101.2, 72.4, 72.1, 70.6, 70.3, 70.2, 70.0, 56.2, 50.5, 50.3, 48.1, 43.2, 40.4, 32.2, 32.2, 23.6, 22.5, 22.4.

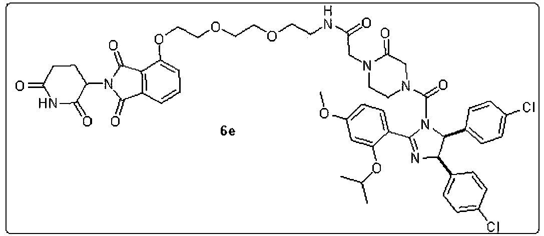

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-(2-((2-(2,6-dioxo-opiperidin-3-yl))-1,3-dioxoisoindolin-4-yl)oxy)ethoxy)ethoxy)eto-yl)acetamide, 6e: The title compound was synthesized using General Method C and then General Method D. The crude product was purified by C-18 silica gel column chromatography on a Biotage system, eluting with acetonitrile/deionized water. The result was secondarily purified by preparative HPLC and the product was obtained as an off-white solid with a yield of 30%.

¹H NMR (600 MHz, CD₃OD) δ 7.79-7.75 (m, 1H), 7.58 (dd, J = 8.4, 2.7 Hz, 1H), 7.48-7.45 (m, 2H), 7.13 (d, J = 8.4 Hz, 2H), 7.07 (dd, J = 8.8, 1.6 Hz, 2H), 7.03 (dd, J = 8.5, 2.3 Hz, 2H), 6.94 (d, J = 8.0 Hz, 2H), 6.66 (dd, J = 8.3, 2.2 Hz, 1H), 6.64 (d, J = 2.3 Hz, 1H), 5.74 (d, J = 10.1 Hz, 1H), 5.56 (d, J = 10.1 Hz, 1H), 5.08 (ddd, J = 12.9, 5.6, 1.5 Hz, 1H), 4.72 (p, J = 6.0 Hz, 1H), 4.61 (bs, 2H), 4.37 (t, J = 4.4 Hz, 2H), 3.92-3.89 (m, 3H), 3.87 (s, 3H), 3.84-3.74 (m, 5H), 3.63-3.60 (m, 2H), 3.52 (t, J = 5.4 Hz, 2H), 3.41-3.32 (m, 2H), 3.07-2.97 (m, 2H), 2.89-2.81 (m, 1H), 2.77-2.64 (m, 2H), 2.11-2.06 (m, 1H), 1.37 (d, J = 6.0 Hz, 3H), 1.34 (d, J = 6.0 Hz, 3H).

¹³C NMR (151 MHz, CD₃OD) δ 174.7, 174.6, 171.5, 169.9, 168.6, 167.4, 165.0, 163.2, 158.6, 157.8, 155.8, 138.0, 137.6, 136.5, 135.2, 134.2, 133.1, 130.7, 130.0, 129.1, 129.0, 120.9, 118.3, 116.8, 113.8, 106.4, 101.2, 72.4, 72.1, 71.3, 70.5, 70.4, 70.1, 56.2, 50.6, 50.5, 50.2, 48.1, 43.2, 40.5, 32.2, 23.7, 22.5, 22.4.

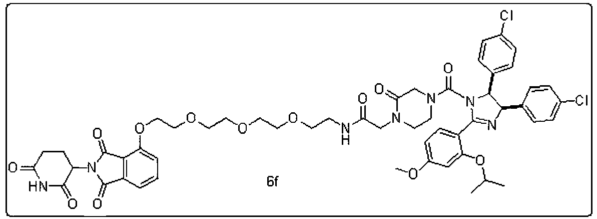

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-(2-((2-(2,6-dioxopiperidin-3-yl))-1,3-dioxo)isoindolin-4-yl)oxy))ethoxy)ethoxy)-ethoxy)ethyl)-acetamide, 6f: The title compound was synthesized using General Method C and then General Method D. The crude product was purified by C-18 silica gel column chromatography on a Biotage system, eluting with acetonitrile/deionized water. The result was secondarily purified by preparative HPLC and the product was obtained as an off-white solid with a yield of 20%.

¹H NMR (600 MHz, CD₃OD) δ 7.77-7.73 (m, 1H), 7.58 (dd, J = 8.4, 1.8 Hz, 1H), 7.47-7.43 (m, 2H), 7.15-7.12 (m, 2H), 7.10-7.07 (m, 2H), 7.04-7.01 (m, 2H), 6.94 (d, J = 8.1 Hz, 2H), 6.67 (dd, J = 8.3, 2.5 Hz, 1H), 6.65 (d, J = 2.3 Hz, 1H), 5.75 (d, J = 10.1 Hz, 1H), 5.56 (d, J = 10.7 Hz, 1H), 5.08 (dd, J = 12.8, 5.4 Hz, 1H), 4.73 (p, J = 6.0 Hz, 1H), 4.39-4.35 (m, 2H), 3.96-3.90 (m, 3H), 3.88 (s, 3H), 3.86-3.75 (m, 5H), 3.67-3.63 (m, 2H), 3.63-3.60 (m, 2H), 3.57-3.54 (m, 2H), 3.49 (t, J = 5.4 Hz, 2H), 3.42-3.35 (m, 1H), 3.34-3.32 (m, 3H), 3.09-2.98 (m, 2H), 2.89-2.81 (m, 1H), 2.77-2.63 (m, 2H), 2.14-2.05 (m, 1H), 1.38 (d, J = 6.0 Hz, 3H), 1.34 (d, J = 6.0 Hz, 3H).

¹³C NMR (151 MHz, CD₃OD) δ 174.6, 171.5, 169.9, 168.6, 167.4, 167.3, 165.0, 163.2, 158.6, 157.8, 155.8, 138.0, 137.6, 136.5, 135.2, 134.2, 133.1, 130.7, 130.0, 129.1, 129.0, 120.9, 118.3, 116.7, 113.8, 106.5, 101.2, 72.4, 72.1, 72.0, 71.6, 71.3, 70.5, 70.5, 70.3, 70.1, 56.2, 50.5, 50.4, 50.2, 48.1, 43.2, 40.4, 32.2, 23.7, 22.5, 22.4.

### Preparation Example 2-2. Synthesis of 10a-c:

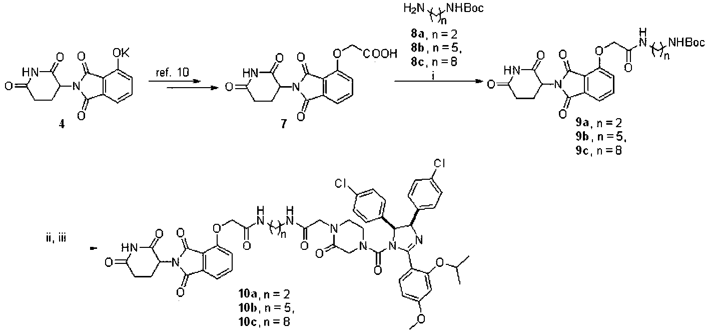

Reagents and conditions: (i) HATU, DIPEA, DMF, rt, 16 h; (ii) TFA, CH₂Cl₂, rt, 1 h; (iii) nutlinic acid, HATU, DIPEA, DMF, rt, 16 h.

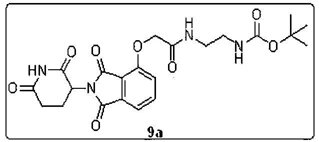

tert-Butyl (2-(2-((2-(2,6-dioxopiperidin-3-yl)-1,3-dioxoisoindolin-4-yl)oxy)acetamide)ethyl)carbamate, 9a: The title compound was synthesized using General Method D. The crude product was purified by silica gel column chromatography on a Biotage system, eluting with methanol/DCM and the product was obtained as a white solid with a yield of 48%.

¹H NMR (600 MHz, DMSO-d6) δ 11.12 (s, 1H), 8.05 (t, J = 5.9 Hz, 1H), 7.81 (dd, J = 8.5, 7.3 Hz, 1H), 7.50 (d, J = 7.2 Hz, 1H), 7.39 (d, J = 8.3 Hz, 1H), 6.87 (t, J = 5.8 Hz, 1H), 5.12 (dd, J = 12.9, 5.5 Hz, 1H), 4.76 (s, 2H), 3.16 (q, J = 6.4 Hz, 2H), 3.01 (q, J = 6.3 Hz, 2H), 2.93-2.83 (m, 1H), 2.63-2.55 (m, 1H), 2.55-2.52 (m, 1H), 2.06-2.00 (m, 1H), 1.36 (s, 9H).

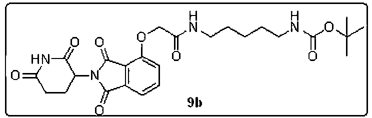

tert-Butyl (5-(2-((2-(2,6-dioxopiperidin-3-yl)-1,3-dioxoisoindolin-4-yl)oxy)acetamide)pentyl)carbamate, 9b: The crude product of the title compound was purified by silica gel column chromatography on a Biotage system eluting with methanol/DCM to obtain the product as a white solid with a yield of 58%.

¹H NMR (600 MHz, DMSO-d6) δ 11.12 (s, 1H), 7.94 (t, J = 5.9 Hz, 1H), 7.82 (dd, J = 8.5, 7.3 Hz, 1H), 7.49 (d, J = 7.2 Hz, 1H), 7.39 (d, J = 8.5 Hz, 1H), 6.77 (t, J = 5.6 Hz, 1H), 5.12 (dd, J = 12.9, 5.5 Hz, 1H), 4.76 (s, 2H), 3.12 (q, J = 6.7 Hz, 2H), 2.91-2.85 (m, 3H), 2.62-2.57 (m, 1H), 2.55-2.52 (m, 1H), 2.07-2.00 (m, 1H), 1.42 (p, J = 7.2 Hz, 2H), 1.38-1.32 (s, 11H), 1.23 (p, J = 7.5 Hz, 2H).

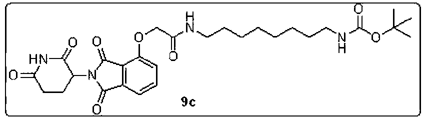

tert-Butyl (8-(2-((2-(2,6-dioxopiperidin-3-yl)-1,3-dioxoisoindolin-4-yl)oxy)acetamide)octyl)carbamate, 9c: The crude product of the title compound was purified by silica gel column chromatography on a Biotage system eluting with methanol/DCM to obtain the product as a white solid with a yield 52%.

¹H NMR (600 MHz, DMSO-*d6*) δ 11.12 (s, 1H), 7.93 (t, J = 5.8 Hz, 1H), 7.83-7.79 (m, 1H), 7.50 (d, J = 7.4 Hz, 1H), 7.39 (d, J = 8.9 Hz, 1H), 6.76 (t, J = 6.0 Hz, 1H), 5.12 (dd, J = 13.0, 5.5 Hz, 1H), 4.76 (s, 2H), 3.13 (q, J = 6.7 Hz, 2H), 2.91-2.85 (m, 3H), 2.63-2.57 (m, 2H), 2.54-2.51(m, 1H), 2.06-2.01 (m, 1H), 1.45-1.39 (m, 2H), 1.38-1.30 (m, 11H), 1.27-1.17 (m, 7H).

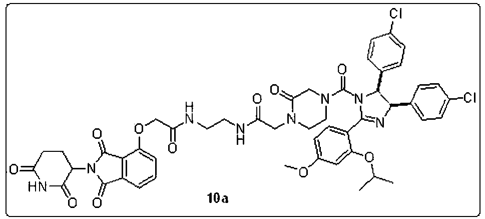

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-((2-(2,6-dioxopiperidin-3-yl)-1,3-dioxoisoindolin-4-yl))oxy)acetamido)ethyl)-acetamide, 10a: The title compound was synthesized using General Method C and then General Method D. The crude product was purified by silica gel column chromatography on a Biotage system, eluting with methanol/DCM. The result was secondarily purified by C-18 silica gel column chromatography on a biotage system, eluting with acetonitrile/deionized water to obtain the product as an off-white solid with a yield of 50%.

¹H NMR (600 MHz, CD₃OD, δ) 7.81 (ddd, *J* = 8.6, 7.3, 1.3 Hz, 1H), 7.57 (dd, *J* = 8.5, 2.5 Hz, 1H), 7.55 (d, *J* = 7.3 Hz, 1H), 7.43 (d, *J* = 8.5 Hz, 1H), 7.15-7.12 (m, 2H), 7.08 (d, *J* = 8.7 Hz, 2H), 7.04-7.01 (m, 2H), 6.95 (d, J = 8.1 Hz, 2H), 6.68-6.64 (m, 2H), 5.74 (dd, *J* = 10.1, 2.9 Hz, 1H), 5.57 (dd, *J* = 10.1, 5.9 Hz, 1H), 5.16 (ddd, *J* = 12.7, 11.6, 5.6 Hz, 1H), 4.76-4.71 (m, 3H), 3.87 (d, *J* = 2.2 Hz, 4H), 3.85-3.71 (m, 3H), 3.53-3.39 (m, 3H), 3.39-3.32 (m, 3H), 3.09-2.96 (m, 2H), 2.92-2.82 (m, 1H), 2.78-2.69 (m, 2H), 2.15-2.09 (m, 1H), 1.38 (d, *J* = 6.0 Hz, 3H), 1.33 (dd, *J* = 6.0, 1.3 Hz, 3H).

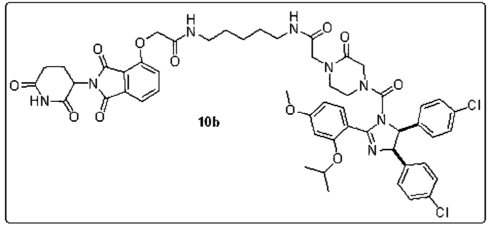

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(5-(2-((2-(2,6-dioxopiperidin-3-yl)-1,3-dioxoisoindolin-4-yl))oxy)acetamido)pentyl-l)acetamide, 10b: The title compound was synthesized using General Method C and then General Method D. The crude product was purified by silica gel column chromatography on a biotage system eluting with methanol/DCM to obtain the product as an off-white solid in 56% yield.

¹H NMR (600 MHz, CD₃OD) δ 7.81 (ddd, J = 8.6, 7.3, 1.4 Hz, 1H), 7.58 (dd, J = 8.3, 2.6 Hz, 1H), 7.54 (dd, J = 7.3, 1.3 Hz, 1H), 7.43 (dd, J = 8.4, 1.3 Hz, 1H), 7.15-7.12 (m, 2H), 7.10-7.07 (m, 2H), 7.05-7.01 (m, 2H), 6.95 (d, J = 8.0 Hz, 2H), 6.68-6.64 (m, 2H), 5.75 (dd, J = 10.1, 2.2 Hz, 1H), 5.57 (d, J = 10.1 Hz, 1H), 5.14 (ddd, J = 12.5, 5.5, 1.1 Hz, 1H), 4.77-4.70 (m, 3H), 3.92-3.74 (m, 5H), 3.82-3.75 (m, 2H), 3.56-3.49 (m, 1H), 3.43-3.34 (m, 1H), 3.37-3.32 (m, 2H), 3.17 (td, J = 7.0, 2.9 Hz, 2H), 3.10-2.99 (m, 2H), 2.92-2.82 (m, 1H), 2.78-2.67 (m, 2H), 2.16-2.09 (m, 1H), 1.58 (p, J = 6.9 Hz, 2H), 1.51 (p, J = 7.2 Hz, 2H), 1.42-1.36 (m, 5H), 1.34 (d, J = 6.0 Hz, 3H).

¹³C NMR (151 MHz, CD₃OD) δ 174.6, 171.4, 169.9, 169.7, 168.3, 167.8, 167.4, 165.0, 163.2, 158.6, 156.3, 155.9, 138.3, 137.6, 136.5, 134.9, 134.2, 133.1, 130.7, 130.0, 129.1, 129.0, 121.8, 119.4, 118.0, 113.8, 106.5, 101.2, 72.4, 72.1, 70.1, 69.5, 56.2, 50.6, 50.3, 48.2, 43.2, 40.3, 40.0, 32.2, 29.9, 29.8, 25.1, 23.6, 22.5, 22.4.

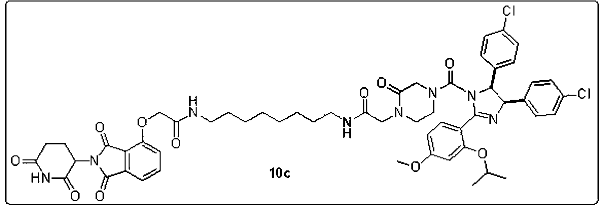

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(8-(2-((2-(2,6-dioxopiperidin-3-yl)-1,3-dioxoisoindolin-4-yl))oxy) acetamido)octyl)- acetamide, 10c: The title compound was synthesized using General Method C and then General Method D. The crude product was purified by silica gel column chromatography on a biotage system, eluting with methanol/DCM to obtain the product as an off-white solid with a yield of 44%.

¹H NMR (600 MHz, CD₃OD) δ 7.78 (dd, J = 8.4, 7.4 Hz, 1H), 7.56 (d, J = 8.4 Hz, 1H), 7.51 (dd, J = 7.2, 1.5 Hz, 1H), 7.40 (d, J = 8.5 Hz, 1H), 7.12-7.09 (m, 2H), 7.07-7.05 (m, 2H), 7.02-6.99 (m, 2H), 6.92 (d, J = 8.0 Hz, 2H), 6.65 (dd, J = 8.3, 2.3 Hz, 1H), 6.63 (d, J = 2.2 Hz, 1H), 5.73 (d, J = 10.1 Hz, 1H), 5.54 (d, J = 10.2 Hz, 1H), 5.11 (dd, J = 12.6, 5.4 Hz, 1H), 4.74-4.68 (m, 3H), 3.88 (d, J = 16.1 Hz, 1H), 3.85 (s, 3H), 3.78 (dd, J = 20.1, 16.7 Hz, 3H), 3.54-3.47 (m, 1H), 3.37 (ddd, J = 9.3, 7.1, 3.7 Hz, 1H), 3.11 (t, J = 7.1 Hz, 2H), 3.08-2.98 (m, 2H), 2.88-2.80 (m, 1H), 2.76-2.66 (m, 2H), 2.14-2.08 (m, 1H), 1.54 (p, J = 7.0 Hz, 2H), 1.43 (p, J = 7.0 Hz, 2H), 1.37-1.22 (m, 16H).

¹³C NMR (151 MHz, CD₃OD) δ 174.6, 171.4, 169.8, 169.7, 168.3, 167.8, 167.4, 165.0, 163.2, 158.6, 156.3, 155.9, 138.2, 137.6, 136.5, 134.9, 134.2, 133.1, 130.7, 130.0, 129.1, 129.0, 121.7, 119.3, 118.0, 113.9, 106.5, 101.3, 72.4, 72.1, 70.1, 69.4, 56.2, 50.6, 50.6, 50.3, 48.2, 43.2, 40.4, 40.1, 32.2, 30.3, 30.2, 27.8, 27.7, 23.6, 22.5, 22.4.

### Preparation Example 2-3. Synthesis of 16a-c

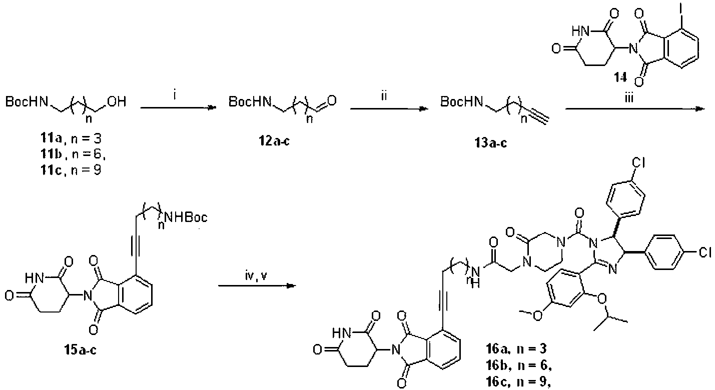

Reagents and conditions: (i) (COCl)₂, DMSO, DIPEA, CH₂Cl₂, -78 °C to -30 °C, 1 h or PCC, Al₂O₃, CH₂Cl₂, rt, 3 h; (ii) Dimethyl (1-diazo-2-oxopropyl)phosphonate, K₂CO₃, CH₃OH, rt, 12 h, (iii) CuI, PdCl₂(PPh₃)₂, Et₃N, DMF, 80°C, 16 h; (iv) TFA, CH₂Cl₂, rt, 1 h; (v) nutlin acid, HATU, DIPEA, DMF, rt, 16 h.

Synthesis of 12a: oxalyl chloride (0.25 g, 1.97 mmol, 2.00 equiv) was added dropwise to a stirred solution of anhydrous DMSO (0.16 g, 2.16 mmol, 2.20 equiv) in anhydrous CH₂Cl₂ (2.00 mL) under a nitrogen atmosphere at -78°C. After stirring for 5 min, a solution of alcohol (0.20 g, 0.98 mmol, 1.00 equiv) in anhydrous CH₂Cl₂ (3.00 mL) was added thereto and the reaction mixture was stirred at -78°C for 15 min. Subsequently, N,N-diisopropylethylamine (0.63 g, 4.91 mmol, 5.00 equiv) was added dropwise thereto. The temperature of the reaction mixture was increased to - 30°C and stirred at the same temperature for 30 min. After completion of the reaction, the reaction mixture was concentrated under reduced pressure. The residue was dissolved in ethyl acetate (15 mL) and washed with water (1 × 15 mL). The organic layer was washed with 5% aqueous NaHCO3 solution (1 × 15 mL), water (1 × 15 mL), and brine (1 × 15 mL), dried over anhydrous Na₂SO₄, filtered, and concentrated under vacuum. The crude product was used in the next step without further purification.

E. General method for the synthesis of compounds 12b to c: pyridinium chlorochromate (1.50 equiv.) was added to a stirred solution of alcohol (1.00 equiv.) in anhydrous CH₂Cl₂. Al₂O₃ (6.00 equiv.) was then added in a portionwise manner over 15 minutes. The reaction mixture was stirred at 25°C for 2 hours. After completion of the reaction, the mixture was filtered through Celite, while washing with CH₂Cl₂. The filtrate was removed under reduced pressure and the residue was dried under high vacuum to obtain the desired product. The crude product was used in the next step without further purification.

F. General method for the synthesis of compounds 13a to c: K₂CO₃ (2.20 equiv.) and dimethyl (1-diazo-2-oxopropyl)phosphonate (1.20 equiv.) were added to a stirred solution of aldehyde (1.00 equiv.) in anhydrous methanol under a nitrogen atmosphere at 25°C. The reaction mixture was stirred at the same temperature for 12 hours. After the reaction was completed, the solvent was removed under reduced pressure and the residue was partitioned between diethyl ether and water. The aqueous layer was extracted with diethyl ether. The combined organic layers were washed with brine, dried over anhydrous Na₂SO₄, filtered, and concentrated under vacuum. The crude product was purified by flash column chromatography on silica gel, eluting with ethyl acetate/hexane, to obtain the desired product with a yield of 40 to 93%.

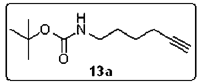

tert-Butyl hexa-5-yn-1-ylcarbamate, 13a: the title compound was synthesized using general method F as a pale yellow liquid with a yield of 40%.

¹H NMR (600 MHz, CDCl₃) δ 4.55 (bs, 1H), 3.13 (t, J = 6.7 Hz, 2H), 2.21 (td, J = 6.8, 2.6 Hz, 2H), 1.94 (t, J = 2.6 Hz, 1H), 1.63-1.52 (m, 4H), 1.43 (s, 9H).

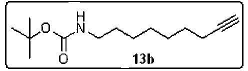

tert-Butyl non-8-yn-1-ylcarbamate, 13b: the title compound was synthesized using general method F as a pale yellow liquid in 93% yield.

¹H NMR (600 MHz, CDCl₃) δ 4.48 (bs, 1H), 3.10 (t, J = 7.1 Hz, 2H), 2.18 (td, J = 7.1, 2.7 Hz, 2H), 1.93 (t, J = 2.6 Hz, 1H), 1.55-1.49 (m, 2H), 1.48-1.42 (s, 11H), 1.42-1.36 (m, 2H), 1.35-1.28 (m, 4H).

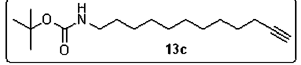

tert-Butyldodeca-11-yn-1-ylcarbamate, 13c: the title compound was synthesized using general method F as a white sticky solid as a yield of 66%.

¹H NMR (600 MHz, CDCl₃) δ 4.46 (bs, 1H), 3.08 (t, J = 7.3 Hz, 2H), 2.17 (td, J = 7.1, 2.6 Hz, 2H), 1.92 (t, J = 2.6 Hz, 1H), 1.54-1.48 (m, 2H), 1.47-1.41 (m, 11H), 1.40-1.33 (m, 2H), 1.27 (m, 10H).

G. General method for the synthesis of 15a-c: alkyne (1.20 equiv), CuI (0.20 equiv), and trimethylamine (1.20 equiv) were added to a stirred solution of 2-(2,6-dioxopiperidin-3-yl)-4-iodoisoindoline-1,3-dione (1.00 equiv) in anhydrous DMF. The reaction mixture was purged with N₂ and PdCl₂(PPh₃)₂ (0.10 equiv) was added thereto. The reaction mixture was stirred at 80°C for 16 hours. After completion of the reaction, the resulting mixture was cooled to room temperature and poured into saturated aqueous NH₄Cl solution. The layers were separated and the aqueous layer was extracted with ethyl acetate. The combined organic layers were washed with saturated aqueous LiCl and brine, and dried over anhydrous Na₂SO₄, filtered, and concentrated under vacuum. The crude product was purified by flash column chromatography on silica gel eluting with acetone/hexane to obtain the desired product with a yield of 50 to 75%.

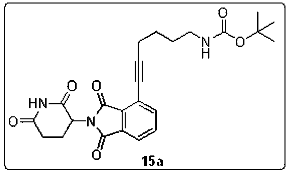

tert-Butyl(6-(2-(2,6-dioxopiperidin-3-yl)-1,3-dioxoisoindolin-4-yl)hexa-5-yn-1-yl)-carbamate, 15a: The title compound was synthesized using General Method G as a white solid with a yield of 50%.

¹H NMR (600 MHz, DMSO-*d₆*) δ 11.07 (s, 1H), 7.83-7.80 (m, 1H), 7.80-7.76 (m, 2H), 6.77 (m, 1H), 5.09 (dd, J = 12.9, 5.5 Hz, 1H), 2.96-2.89 (m, 2H), 2.85 (ddd, J = 17.2, 14.0, 5.5 Hz, 1H), 2.59-2.54 (m, 2H), 2.53-2.47 (m, 4H), 2.02 (m, 1H), 1.33 (s, 9H).

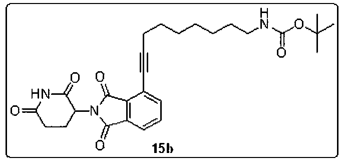

tert-Butyl (9-(2-(2,6-dioxopiperidin-3-yl)-1,3-dioxoisoindolin-4-yl)non-8-yn-1-yl) carbamate, 15b: The title compound was synthesized as a yellow solid with a yield of 64% using general method G.

¹H NMR (600 MHz, DMSO-*d₆*) δ 11.09 (s, 1H), 7.82 (dd, J = 6.2, 2.2 Hz, 1H), 7.80-7.75 (m, 2H), 6.71 (t, J = 5.5 Hz, 1H), 5.10 (dd, J = 12.9, 5.5 Hz, 1H), 2.89-2.81 (m, 3H), 2.60-2.54 (m, 1H), 2.53-2.48 (m, 1H), 2.05-1.99 (m, 1H), 1.54 (p, J = 6 Hz, 2H), 1.45-1.38 (m, 2H), 1.36-1.16 (m, 17H).

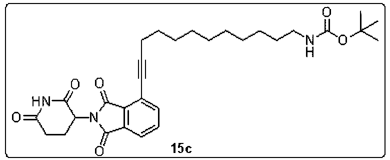

tert-Butyl(12-(2-(2,6-dioxopiperidin-3-yl)-1,3-dioxoisoindolin-4-yl)dodeca-11-yn-1-yl)carbamate, 15c: The title compound was synthesized as a yellow solid with a yield of 75% using general method G.

¹H NMR (600 MHz, DMSO-*d₆*) δ 11.09 (s, 1H), 7.82 (dd, J = 6.6, 1.9 Hz, 1H), 7.80-7.75 (m, 2H), 6.70 (t, J = 6 Hz, 1H), 5.10 (dd, J = 12.9, 5.5 Hz, 1H), 2.89-2.81 (m, 3H), 2.59-2.53 (m, 1H), 2.53-2.48 (m, 1H), 2.04-1.98 (m, 1H), 1.54 (p, J = 6 Hz, 2H), 1.42 (p, J = 6 Hz, 2H), 1.34-1.13 (m, 23H).

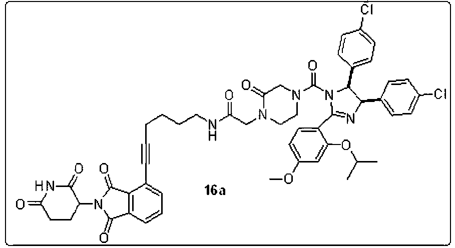

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(6-(2-(2,6-dioxopipe-lysin-3-yl)-1,3-dioxoisoindolin-4-yl)hexa-5-yn-1-yl) acetamide, 16a: The title compound was synthesized using General Method C and then General Method D. The crude product was purified by silica gel column chromatography on a Biotage system eluting with methanol/DCM. The result was secondarily purified by biotage on C-18 silica gel column chromatography eluting with acetonitrile/deionized water to obtain the product as an off-white solid with a yield of 35%.

¹H NMR (600 MHz, CD₃OD) δ 7.83-7.78 (m, 1H), 7.77-7.72 (m, 2H), 7.58 (dd, J = 8.4, 3.7 Hz, 1H), 7.15-7.12 (m, 2H), 7.08 (d, J = 8.7 Hz, 2H), 7.05-7.01 (m, 2H), 6.95 (d, J = 7.8 Hz, 2H), 6.69-6.64 (m, 2H), 5.75 (d, J = 10.2 Hz, 1H), 5.57 (d, J = 10.1 Hz, 1H), 5.16 (ddd, J = 12.5, 5.4, 2.8 Hz, 1H), 4.73 (p, J = 6.0 Hz, 1H), 3.99-3.77 (m, 7H), 3.56-3.49 (m, 1H), 3.45-3.35 (m, 1H), 3.26 (t, J = 6.7 Hz, 2H), 3.10-3.02 (m, 2H), 2.91-2.81 (m, 1H), 2.79-2.69 (m, 2H), 2.55 (t, J = 6.7 Hz, 2H), 2.16-2.08 (m, 1H), 1.79-1.72 (m, 2H), 1.71-1.63 (m, 2H), 1.38 (d, J = 6.0 Hz, 3H), 1.34 (dd, J = 6.0, 1.4 Hz, 3H).

¹³C NMR (151 MHz, CD₃OD) δ 174.6, 171.5, 169.8, 168.0, 167.6, 167.3, 165.0, 163.2, 158.6, 155.8, 139.5, 137.5, 136.4, 135.3, 134.2, 133.8, 133.1, 131.9, 130.7, 130.0, 129.1, 129.0, 123.4, 122.4, 113.7, 106.5, 101.3, 99.6, 77.4, 72.4, 72.0, 70.1, 56.2, 50.6, 50.3, 48.1, 43.2, 39.9, 32.2, 29.4, 26.6, 23.6, 22.5, 22.4, 20.0.

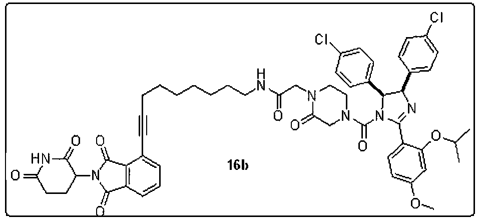

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(9-(2-(2,6-dioxopiperidin-3-yl)-1,3-dioxoisoindolin-4-yl)non-8-yn-1-yl) acetamide, 16b: The title compound was synthesized using General Method C and then General Method D. The crude product was purified by silica gel column chromatography on a Biotage system, eluting with methanol/DCM. The result was secondarily purified by biotage on C-18 silica gel column chromatography, eluting with acetonitrile/deionized water, to obtain the product as an off-white solid with a yield of 28%.

¹H NMR (600 MHz, CD₃OD) δ 7.80 (dd, J = 6.4, 2.1 Hz, 1H), 7.76-7.72 (m, 2H), 7.58 (dd, J = 8.4, 2.2 Hz, 1H), 7.14 (d, J = 8.4 Hz, 2H), 7.08 (d, J = 8.8 Hz, 2H), 7.03 (d, J = 7.1 Hz, 2H), 6.94 (d, J = 8.0 Hz, 2H), 6.67 (ddd, J = 8.4, 2.3, 0.9 Hz, 1H), 6.65 (d, J = 2.2 Hz, 1H), 5.75 (d, J = 10.1 Hz, 1H), 5.56 (d, J = 10.1 Hz, 1H), 5.14 (dd, J = 12.7, 5.5 Hz, 1H), 4.73 (p, J = 6.0 Hz, 1H), 3.90 (dd, J = 16.1, 3.1 Hz, 1H), 3.88 (s, 3H), 3.85-3.76 (m, 3H), 3.55-3.49 (m, 1H), 3.39 (m, 1H), 3.16 (t, J = 7.0 Hz, 2H), 3.10-2.99 (m, 2H), 2.91-2.83 (m, 1H), 2.78-2.67 (m, 2H), 2.52 (t, J = 6.8 Hz, 2H), 2.13 (m, 1H), 1.68-1.62 (m, 2H), 1.59-1.53 (m, 2H), 1.50 (p, J = 7.1 Hz, 2H), 1.40-1.32 (m, 10H).

¹³C NMR (151 MHz, CD₃OD) δ 174.6, 171.5, 169.7, 168.1, 167.5, 167.4, 165.0, 163.2, 158.6, 155.8, 139.5, 137.6, 136.5, 135.3, 134.2, 133.1, 131.9, 130.7, 130.0, 129.1, 129.0, 123.3, 122.6, 113.8, 106.5, 101.3, 100.1, 77.1, 72.5, 72.1, 70.1, 56.2, 50.6, 50.2, 48.2, 43.2, 40.4, 32.2, 30.2, 29.8, 29.6, 29.3, 27.8, 23.6, 22.5, 22.4, 20.3.

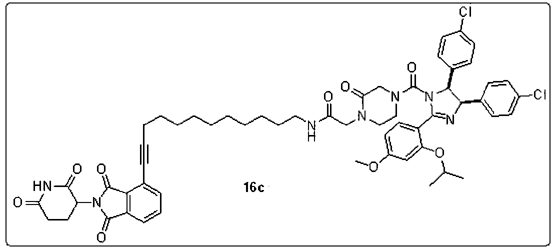

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(12-(2-(2,6-dioxopiperidin-3-yl)-1,3-dioxoisoindolin-4-yl) dodeca-11-yn-1-yl) acetamide, 16c: The title compound was synthesized using General Method C and then General Method D. The crude product was purified by silica gel column chromatography on a Biotage system, eluting with methanol/DCM. The result was secondarily purified by biotage on a C-18 silica gel column chromatography eluting with acetonitrile/deionized water to obtain the product as an off-white solid with a yield of 42%.

¹H NMR (600 MHz, CD₃OD) δ 7.77 (dt, J = 6.3, 2.1 Hz, 1H), 7.74-7.69 (m, 2H), 7.56 (d, J = 8.4 Hz, 1H), 7.13-7.10 (m, 2H), 7.07-7.05 (m, 2H), 7.02-6.99 (m, 2H), 6.92 (d, J = 8.0 Hz, 2H), 6.65 (dd, J = 8.4, 2.3 Hz, 1H), 6.63 (d, J = 2.3 Hz, 1H), 5.73 (d, J = 10.1 Hz, 1H), 5.54 (d, J = 10.1 Hz, 1H), 5.11 (dd, J = 12.6, 5.5 Hz, 1H), 4.71 (p, J = 6.0 Hz, 1H), 3.88 (d, J = 16.1 Hz, 1H), 3.85 (s, 3H), 3.84-3.74 (m, 3H), 3.50 (dt, J = 13.7, 5.2 Hz, 1H), 3.41-3.34 (m, 1H), 3.11 (t, J = 7.1 Hz, 2H), 3.07-2.98 (m, 2H), 2.88-2.80 (m, 1H), 2.76-2.65 (m, 2H), 2.49 (t, J = 6.9 Hz, 2H), 2.13-2.07 (m, 1H), 1.66-1.59 (m, 2H), 1.55-1.49 (m, 2H), 1.47-1.39 (m, 2H), 1.38-1.25 (m, 16H).

¹³C NMR (151 MHz, CD₃OD) δ 174.6, 171.4, 169.6, 168.1, 167.5, 167.4, 165.0, 163.2, 158.6, 155.8, 139.5, 137.6, 136.4, 135.3, 134.2, 133.8, 133.1, 131.9, 130.7, 130.0, 129.1, 129.0, 123.3, 122.6, 113.8, 106.5, 101.2, 100.2, 77.1, 72.4, 72.0, 70.1, 56.2, 50.6, 50.2, 48.2, 43.2, 40.4, 32.2, 30.5, 30.5, 30.4, 30.3, 30.2, 29.8, 29.4, 27.9, 23.6, 22.5, 22.4, 20.4.

### Preparation Example 2-4. Synthesis of Compounds 17 to 21

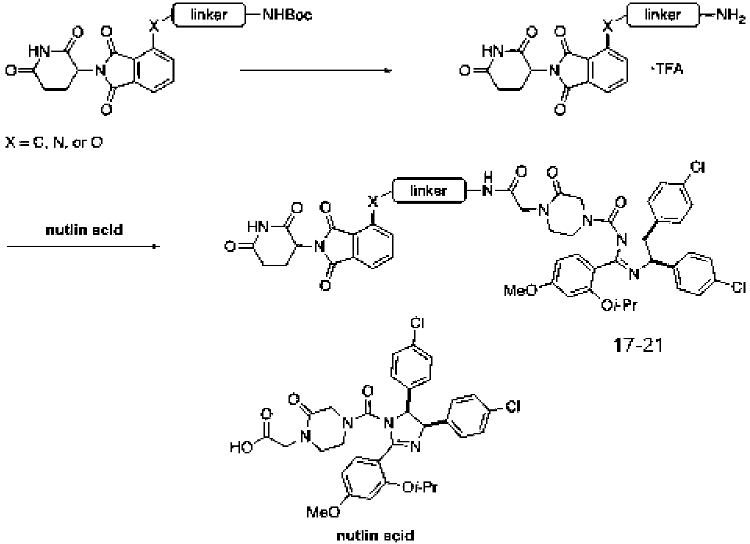

### Preparation Example 2-4-1. Synthesis of Compound 17 (2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(8-((2-(2,6-dioxopiperidin-3-yl)-1,3-dioxoisoindolin-4-yl)amino)octyl)acetamide)

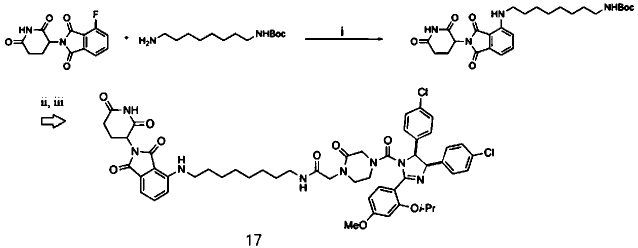

Reagents and conditions: (i) DIPEA, NMP, 90°C, 17 h; (ii) TFA, CH₂Cl₂, rt, 1 h; (iii) Nutlin acid, HATU, DIPEA, DMF, rt, 16 h.

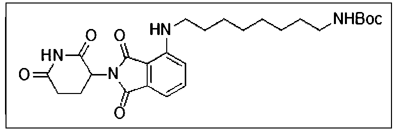

tert-Butyl (8-((2-(2,6-dioxopiperidin-3-yl)-1,3-dioxoisoindolin-4-yl)amino)octyl)carbamate: DIPEA (2.00 equiv) was added to a stirred solution of 2-(2,6-dioxopiperidin-3-yl)-4-fluoroisoindoline-1,3-dione (1.00 equiv) and tert-butyl (8-aminooctyl)carbamate (1.10 equiv) in anhydrous NMP. The reaction mixture was stirred at 90°C for 17 hours. After completion of the reaction, the mixture was diluted with water and ethyl acetate and extracted with ethyl acetate. The combined organic layers were washed with 10% citric acid, aqueous NaHCO₃, water, and brine, dried over Na₂SO₄, filtered, and concentrated under reduced pressure. The product was purified by flash column chromatography on silica gel (30% acetone/hexane) to obtain the desired product with a yield of 64%.

¹H NMR (600 MHz, CD₃OD) δ 7.55 (dd, J = 8.6, 7.1 Hz, 1H), 7.04 (dd, J = 7.8, 2.4 Hz, 2H), 5.06 (dd, J = 12.5, 5.5 Hz, 1H), 3.34 (p, J = 1.7) Hz, 2H), 3.29 (p, J = 1.6 Hz, 1H), 3.01 (t, J = 7.1 Hz, 2H), 2.90-2.82 (m, 1H), 2.77-2.67 (m, 2H), 2.14-2.08 (m, 1H), 1.67 (p, J = 7.1 Hz, 2H), 1.50-1.30 (m, 18H).

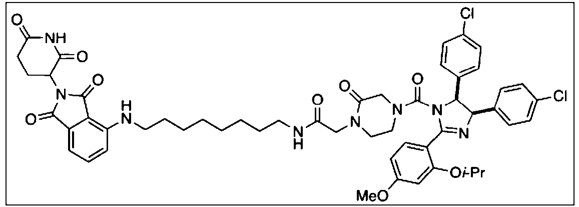

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(8-((2-(2,6-dioxopiperidin-3-yl)-1,3-dioxoisoindolin-4-yl)amino)octyl)acetamide: The title compound was synthesized using General Method C and then General Method D and obtained as a yellow solid with a yield of 51%.

¹H NMR (600 MHz, CD₃OD) δ 7.94 (t, J = 5.7 Hz, 1H), 7.60 (dd, J = 8.4, 1.5 Hz, 1H), 7.55 (dd, J = 8.6, 7.2 Hz, 1H), 7.21-7.13 (m, 2H), 7.13-7.08 (m, 2H), 7.07-7.01 (m, 4H), 6.96 (d, J = 8.1 Hz, 2H), 6.73-6.67 (m, 2H), 5.83 (d, J = 10.2 Hz, 1H), 5.63 (d, J = 10.3 Hz, 1H), 5.05 (dd, J = 12.5, 5.5 Hz, 1H), 4.80-4.73 (m, 1H), 3.96-3.76 (m, 7H), 3.60-3.49 (m, 1H), 3.45-3.39 (m, 1H), 3.34-3.32 (m, 2H), 3.18-3.13 (m, 2H), 3.11-3.01 (m, 2H), 2.90-2.80 (m, 1H), 2.78-2.66 (m, 2H), 2.13-2.06 (m, 1H), 1.66 (p, J = 7.1 Hz, 2H), 1.57-1.24 (m, 16H). LRMS (ESI) m/z ([M+H]+) cacld for C 53H 59Cl 2N 8O 9: 1021.4, found: 1021.3.

### Preparation Example 2-4-2. Synthesis of Compound 18 (2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(4-(4-(((2-(2,6-dioxopiperidin-3-yl)-1,3-dioxoisoindolin-4-yl)oxy)methyl)phenyl)but-3-yn-1-yl)acetamide)

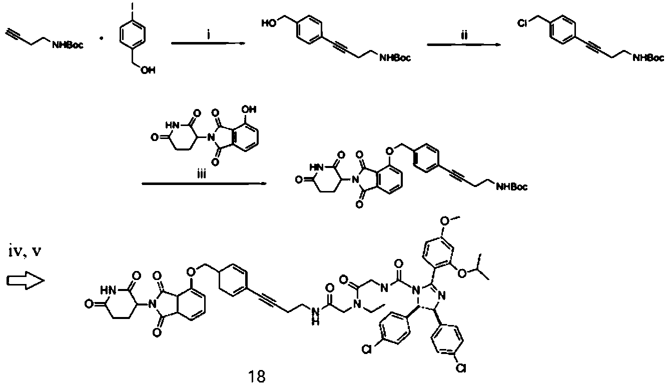

Reagents and conditions: (i) CuI, PdCl₂(PPh₃)₂, Et3N, DMF, 80°C, 3 h; (ii) MsCl, NEt₃, CH₂Cl₂, 0°C ->rt, 25 h; (iii) K₂CO₃, DMF, 60°C, 4 h; (iv) TFA, CH₂Cl₂, rt, 1 h; (v) nutlin acid, HATU, DIPEA, DMF, rt, 16 h.

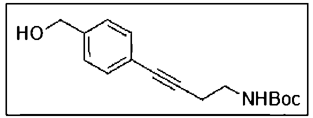

tert-Butyl (4-(4-(hydroxymethyl)phenyl)but-3-yn-1-yl)carbamate: CuI (0.20 equiv) and Et₃N (1.20 equiv) were added to a stirred solution of (4-iodophenyl) methanol (1.00 equiv) dissolved in anhydrous DMF. The reaction mixture was purged with N₂, and PdCl₂(PPh₃)₂ (0.10 equiv) was added thereto. The reaction mixture was stirred at 80°C for 16 hours. After the reaction was completed, the reaction mixture was cooled to room temperature and poured into a saturated aqueous NH₄Cl solution. The resulting mixture was extracted with ethyl acetate. The combined organic layers were washed with a saturated aqueous LiCl solution and brine, dried over anhydrous Na₂SO₄, filtered, and concentrated under reduced pressure. The product was purified by column chromatography on C-18 silica gel (10%→100% acetonitrile/water containing 0.1% formic acid) to obtain the desired product with a yield of 34%.

¹H NMR (600 MHz, CDCl₃) δ 7.42-7.38 (m, 2H), 7.31-7.28 (m, 2H), 4.87 (s, 1H), 4.69 (s, 2H), 3.36 (d, J = 7.3 Hz, 2H), 2.60 (t, J = 6.5 Hz, 2H), 1.46 (s, 9H) .

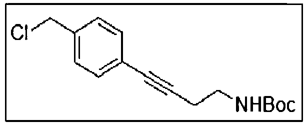

tert-Butyl(4-(4-(chloromethyl)phenyl)but-3-yn-1-yl)carbamate: tert-butyl(4-(4-(hydroxymethyl)phenyl)but-3-yn-1-yl)carbamate (1.00 equiv) was dissolved in CH₂Cl₂ under a N₂ atmosphere and cooled to 0°C. MsCl (1.20 equiv) and Et₃N (1.50 equiv) were added thereto and the reaction mixture was stirred for 2 hours and warmed to room temperature. After the reaction was completed, the mixture was diluted with water and CH₂Cl₂ and extracted with CH₂Cl₂. The combined organic layers were dried over MgSO₄, filtered, and concentrated under reduced pressure. The product was purified by column chromatography on silica gel (0%→100% ethyl acetate/hexane) to obtain the desired product with a yield of 63%.

¹H NMR (600 MHz, CDCl₃) δ 7.39-7.36 (m, 2H), 7.32-7.29 (m, 2H), 4.84 (s, 1H), 4.55 (s, 2H), 3.35 (d, J = 6.5 Hz, 2H), 2.59 (t, J = 6.5 Hz, 2H), 1.44 (s, 9H).

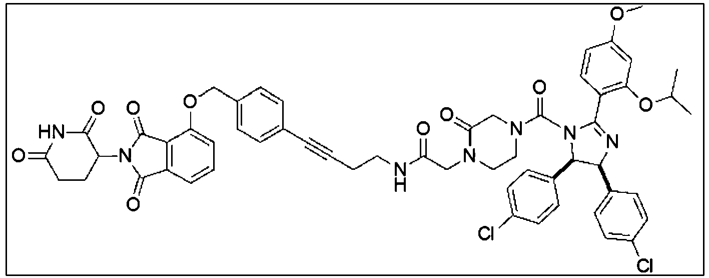

2-(4-(cis-4,5-bis(4-chlorophenyl)-2-(2-isopropoxy-4-methoxyphenyl)-4,5-dihydro-1H-imidazole-1-carbonyl)- 2-oxopiperazin-1-yl)-N-(4-(4-(((2-(2,6-dioxopiperidin-3-yl)-1,3-dioxoisoindolin-4-yl)oxy)methyl)phenyl)but-3-yn-1-yl)acetamide. The title compound was synthesized using General Method C and then General Method D and was obtained as a white solid with a yield of 46%.

¹H NMR (600 MHz, CD₃OD) δ 7.74 (ddd, J = 8.7, 7.3, 1.4 Hz, 1H), 7.56 (d, J = 8.2 Hz, 1H), 7.51-7.43 (m, 4H), 7.42-7.39 (m, 2H), 7.14-7.11 (m, 2H), 7.09-7.06 (m, 2H), 7.02 (d, J = 8.2 Hz, 2H), 6.94 (d, J = 8.1 Hz, 2H), 6.68-6.64 (m, 2H), 5.74 (d, J = 10.1 Hz, 1H), 5.55 (d, J = 10.1 Hz, 1H), 5.35 (s, 2H), 5.12 (dd, J = 12.8, 5.5 Hz, 1H), 4.72 (p, J = 6.0 Hz, 1H), 3.97 (dd, J = 16.2, 1.8 Hz, 1H), 3.92-3.74 (m, 6H), 3.49-3.36 (m, 3H), 3.03-2.96 (m, 2H), 2.90-2.81 (m, 1H), 2.77-2.68 (m, 2H), 2.61 (t, J = 6.7 Hz, 2H), 2.15-2.08 (m, 1H), 1.37-1.32 (m, 6H).

LRMS (ESI) *m*/*z* ([M+H]⁺) cacld for C₅₆H₅₁Cl₂N₇O₁₀: 1052.3, found:1052.3.

### Preparation Example 2-4-3. Synthesis of Compound 19 (2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(4-(4-(((2-(2,6-dioxopiperidin-3-yl)-1,3-dioxoisoindolin-4-yl)oxy)methyl)phenyl) butyl) acetamide

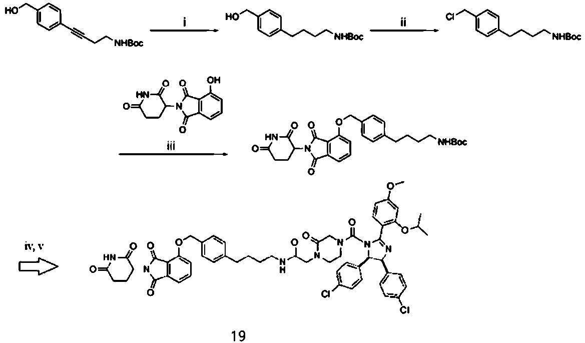

Reagents and conditions: (i) H₂, Pd/C, ethyl acetate, rt, 2 h; (ii) MsCl, NEt₃, CH₂Cl₂, 0°C->rt, 20 h; (iii) K₂CO₃, DMF, 60°C, 4 h; (iv) TFA, CH₂Cl₂, rt, 1 h; (v) nutlin acid, HATU, DIPEA, DMF, rt, 16 h.

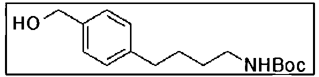

tert-Butyl (4-(4-(hydroxymethyl)phenyl)butyl)carbamate: 10% Pd/C (0.05 eq) was added to a stirred solution of tert-butyl (4-(4-(hydroxymethyl)phenyl)but-3-yn-1-yl)carbamate (1.00 eq) in ethyl acetate. The reaction mixture was hydrogenated at room temperature for 2 hours. After completion of the reaction, the catalyst was removed by filtration through a pad of Celite and then washed with ethyl acetate. The product was purified by column chromatography on C-18 silica gel (10%→100% acetonitrile/water containing 0.1% formic acid) to obtain a product with a yield of 78%.

¹H NMR (600 MHz, DMSO-*d₆*) δ 7.20 (d, J = 7.9 Hz, 2H), 7.14-7.11 (m, 2H), 6.80 (t, J = 5.8 Hz, 1H), 5.08 (s, 1H), 4.44 (d, J = 3.4 Hz, 2H), 2.92 (q, J = 6.7 Hz, 2H), 2.55-2.51 (m, 2H), 1.51 (p, J = 7.9 Hz, 2H), 1.36 (s, 11H).

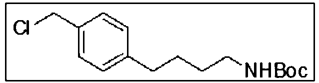

tert-Butyl(4-(4-(chloromethyl)phenyl)butyl)carbamate: tert-butyl(4-(4-(hydroxymethyl)phenyl)butyl)carbamate (1.00 equiv) was dissolved in CH₂Cl₂ under N₂ atmosphere and cooled to 0° C. MsCl (1.20 equiv) and Et₃N (1.50 equiv) were added thereto and the reaction mixture was stirred for 20 hours and warmed to room temperature. After the reaction was completed, the mixture was diluted with water and CH₂Cl₂ and extracted with CH₂Cl₂. The combined organic layers were dried over MgSO₄, filtered, and concentrated under reduced pressure. The result was purified by column chromatography on silica gel (0%→100% ethyl acetate/hexane) to obtain a product with a yield of 50%.

¹H NMR (600 MHz, DMSO-*d₆*) δ 7.35-7.31 (m, 2H), 7.21-7.17 (m, 2H), 6.80 (t, J = 5.8 Hz, 1H), 4.72 (s, 2H), 2.92 (q, J = 6.7 Hz, 2H), 2.56 (t, J = 7.7 Hz, 2H), 1.52 (p, J = 7.7 Hz, 2H), 1.36 (s, 11H).

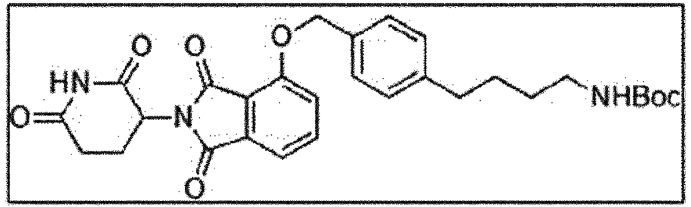

4-((4-(4-aminobutyl)benzyl)oxy)-2-(2,6-dioxopiperidin-3-yl)isoindoline-1,3-dione: K₂CO₃ (2.50 equiv) was added to a stirred solution of tert-butyl (4-(4-(chloromethyl)phenyl)butyl)carbamate (1.11 equiv) and 2-(2,6-dioxopiperidin-3-yl)-4-hydroxyisoindoline-1,3-dione (1.00 equiv) in anhydrous DMF. The reaction mixture was stirred at 60°C for 6 hours. After the reaction was complete, the mixture was diluted with water and CH₂Cl₂. The combined organic layers were dried over MgSO₄, filtered, and concentrated under reduced pressure. The result was purified by column chromatography on C-18 silica gel (10%→100% acetonitrile/water containing 0.1% formic acid) to obtain a product with a yield of 44%.

¹H NMR (600 MHz, DMSO-*d₆*) δ 11.12 (s, 1H), 7.82 (dd, J = 8.5, 7.3 Hz, 1H), 7.59 (d, J = 8.6 Hz, 1H), 7.46 (d, J = 7.2 Hz, 1H), 7.40 (d, J = 7.9 Hz, 2H), 7.23 (d, J = 8.0 Hz, 2H), 6.80 (t, J = 5.8 Hz, 1H), 5.32 (s, 2H), 5.09 (dd, J = 12.9, 5.4 Hz, 1H), 2.95-2.83 (m, 4H), 2.61-2.51 (m, 4H), 2.06-2.00 (m, 1H).

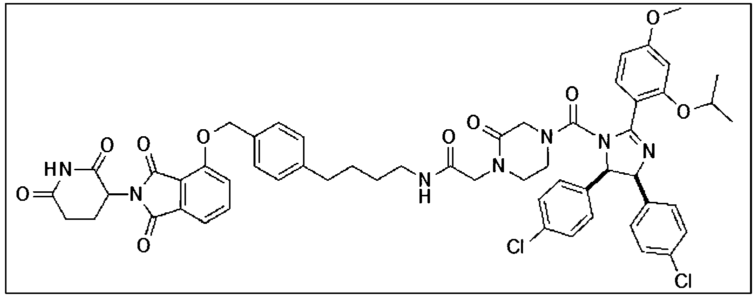

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(4-(4-(((2-(2,6-dioxopiperidin-3-yl)-1,3-dioxoisoindolin-4-yl)oxy)methyl)phenyl) butyl)acetamide. The title compound was synthesized using General Method C and then General Method D and was obtained as a white solid with a yield of 49%.

¹H NMR (600 MHz, CD₃OD) δ 7.75-7.71 (m, 1H), 7.58 (dd, J = 8.3, 2.8 Hz, 1H), 7.49-7.39 (m, 4H), 7.23-7.19 (m, 2H), 7.16-7.12 (m, 2H), 7.10-7.06 (m, 2H), 7.05-7.01 (m, 2H), 6.94 (d, J = 8.0 Hz, 2H), 6.68-6.63 (m, 2H), 5.75 (d, J = 10.2 Hz, 1H), 5.57 (d, J = 10.1 Hz, 1H), 5.32 (s, 2H), 5.10 (dd, J = 12.5, 5.5 Hz, 1H), 4.73 (p, J = 6.0 Hz, 1H), 3.93-3.73 (m, 7H), 3.55-3.48 (m, 1H), 3.43-3.35 (m, 1H), 3.18 (t, J = 6.9 Hz, 2H), 3.08-2.96 (m, 2H), 2.89-2.81 (m, 1H), 2.77-2.67 (m, 2H), 2.63 (t, J = 7.5 Hz, 2H), 2.14-2.07 (m, 1H), 1.66-1.58 (m, 2H), 1.50 (p, J = 7.0 Hz, 2H), 1.39-1.32 (m, 6H).

LRMS (ESI) *m*/*z* ([M+H]⁺) cacld for C₅₆H₅₅Cl₂N₇O₁₀: 1056.4, found: 1056.4.

### Preparation Example 2-4-4. Synthesis of Compound 20 (2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(9-(2-(2,6-dioxopiperidin-3-yl)-1,3-dioxoisoindolin-4-yl)nonyl)acetamide)

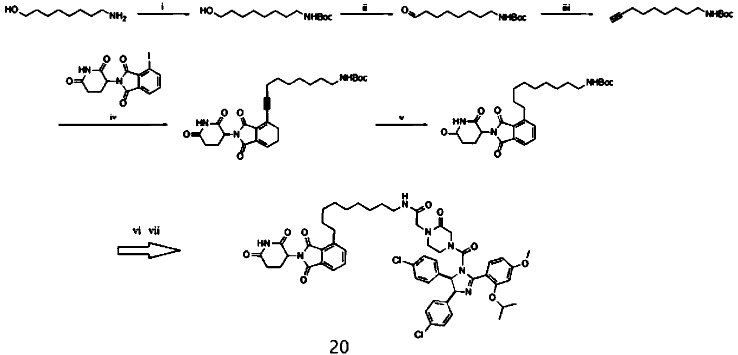

Reagents and conditions: (i) (Boc) 2O, CH₂Cl₂, 0° C->rt, 3 h; (ii) PCC, Al₂O₃, CH₂Cl₂, rt, 3 h; (iii) dimethyl (1-diazo-2-oxopropyl)phosphonate, K₂CO₃, CH₃OH, rt, 4 h; (iv) CuI, PdCl₂(PPh₃)₂, Et₃N, DMF, 80°C; (v) H 2, Pd/C, ethanol, rt, 63 h; (vi) TFA, CH₂Cl₂, rt, 1 h; (vii) nutlin acid, HATU, DIPEA, DMF, rt, 16 h.

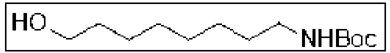

tert-Butyl(8-hydroxyoctyl)carbamate: (Boc)₂O (1.20 equiv.) was added to a stirred solution of 8-aminooctan-1-ol (1.00 equiv.) in anhydrous CH₂Cl₂ at 0°C and the reaction mixture was stirred at room temperature for 3 hours. After the reaction was completed, the solvent was concentrated under reduced pressure. The result was purified by silica gel column chromatography (40% ethyl acetate/hexane) to obtain a product with a yield of 32%.

¹H NMR (600 MHz, CDCl₃) δ 4.49 (s, 1H), 3.64 (t, J = 6.6 Hz, 2H), 3.16-3.01 (m, 2H), 1.55 (dt, J = 8.0, 6.7 Hz, 3H), 1.44 (s, 11H), 1.38-1.28 (m, 8H).

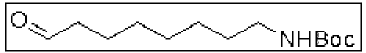

tert-Butyl(8-oxooctyl)carbamate. Al₂O₃ (6.00 equiv.) was added to a stirred mixture of tert-butyl(8-hydroxyoctyl)carbamate (1.00 equiv.) and PCC (1.50 equiv.) in anhydrous CH₂Cl₂ and the mixture was stirred at room temperature under N₂ atmosphere for 3 hours. After completion of the reaction, the reaction mixture was filtered over silica and the solvent was concentrated under reduced pressure. The mixture was used in the next step without further purification.

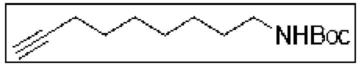

tert-Butyl (8-oxooctyl)carbamate. K₂CO₃ (2.20 equiv) and dimethyl (1-diazo-2-oxopropyl)phosphonate (1.20 equiv) were added to a stirred mixture of tert-butyl bi-8-yn-1-ylcarbamate in anhydrous CH₃OH and the mixture was stirred at room temperature for 4 hours. After completion of the reaction, the reaction mixture was diluted with water and extracted with diethyl ether. The combined organic layers were washed with brine, dried over Na₂SO₄, filtered, and concentrated under reduced pressure. The mixture was used in the next step without further purification.

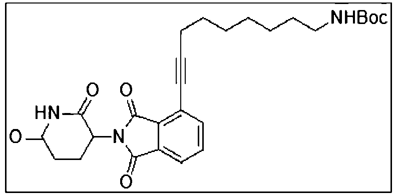

tert-Butyl (9-(2-(2,6-dioxopiperidin-3-yl)-1,3-dioxoisoindolin-4-yl)non-8-yn-1-yl)carbamate. tert-Butyl bi-8-yn-1-ylcarbamate (1.20 equiv), CuI (0.20 equiv), and Et₃N (1.20 equiv) were added to a stirred solution of 2-(2,6-dioxopiperidin-3-yl)-4-iodoisoindolin-1,3-dione in anhydrous DMF. The reaction mixture was stirred at 80°C for 3 hours. After completion of the reaction, the reaction mixture was cooled to room temperature and poured into a saturated aqueous solution of NH₄Cl. The resulting mixture was extracted with ethyl acetate. The combined organic layers were washed with saturated aqueous brine, dried over anhydrous Na₂SO₄, filtered, and concentrated under reduced pressure. The result was purified by column chromatography on silica gel (40% acetone/hexane) to obtain a product with a yield of 54%.

¹H NMR (600 MHz, CDCl₃) δ 8.36 (s, 1H), 7.77 (dd, J = 7.3, 1.2 Hz, 1H), 7.69 (dd, J = 7.9, 1.2 Hz, 1H), 7.67-7.64 (m, 1H), 4.99 (dd, J = 12.5, 5.4 Hz, 1H), 4.60 (s, 1H), 3.11 (d, J = 6.3 Hz, 2H), 2.94-2.71 (m, 3H), 2.52 (t, J = 6.9 Hz, 2H), 2.16-2.11 (m, 1H), 1.65 (p, J = 6.5 Hz, 2H), 1.44 (s, 13H), 1.35 (dd, J = 8.0, 4.6 Hz, 4H).

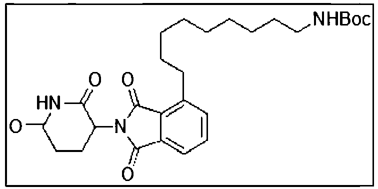

tert-Butyl (9-(2-(2,6-dioxopiperidin-3-yl)-1,3-dioxoisoindolin-4-yl)nonyl)carbamate. 10% Pd/C (0.01 equiv.) was added to a stirred solution of tert-butyl (9-(2-(2,6-dioxopiperidin-3-yl)-1,3-dioxoisoindolin-4-yl)non-8-yn-1-yl)carbamate (1.00 equiv.) in ethanol. The reaction mixture was hydrogenated at 40°C for 63 hours. The catalyst was removed by filtration through a pad of Celite, and then rinsed with ethyl acetate. The result was purified by column chromatography on C-18 silica gel (10%-100% acetonitrile/water containing 0.1% formic acid) to obtain a product with a yield of 54%.

¹H NMR (600 MHz, CDCl₃) δ 8.11 (s, 1H), 7.72 (dd, J = 7.4, 1.0 Hz, 1H), 7.63 (t, J = 7.5 Hz, 1H), 7.52 (dd, J = 7.7, 1.0 Hz, 1H), 4.98 (dd, J = 12.6, 5.4 Hz, 1H), 4.53 (s, 1H), 3.15-2.99 (m, 4H), 2.95-2.70 (m, 3H), 2.19-2.11 (m, 1H), 1.64 (p, J = 7.7 Hz, 2H), 1.49-1.27 (m, 21H).

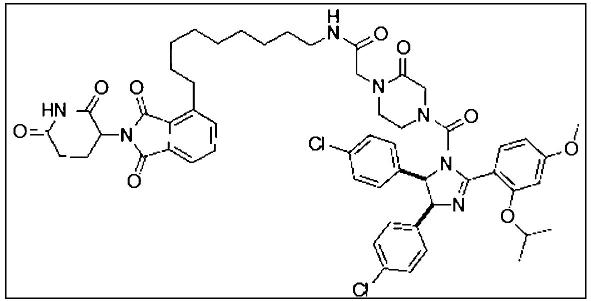

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(9-(2-(2,6-dioxopiperidin-3-yl)-1,3-dioxoisoindolin-4-yl)nonyl)acetamide. The title compound was synthesized using General Method C and then General Method D, and was obtained as a white solid with a yield of 51%.

¹H NMR (600 MHz, CD₃OD) δ 7.95 (t, J = 5.7 Hz, 1H), 7.74-7.69 (m, 2H), 7.64-7.58 (m, 2H), 7.17-7.13 (m, 2H), 7.12-7.07 (m, 2H), 7.06-7.01 (m, 2H), 6.95 (d, J = 8.0 Hz, 2H), 6.70-6.66 (m, 2H), 5.79 (d, J = 10.2 Hz, 1H), 5.60 (d, J = 10.2 Hz, 1H), 5.13 (dd, J = 12.7, 5.5 Hz, 1H), 4.76 (p, J = 6.0 Hz, 1H), 3.96-3.74 (m, 6H), 3.54 (d, J = 13.6 Hz, 1H), 3.44-3.38 (m, 1H), 3.15 (t, J = 6.9 Hz, 2H), 3.12-3.00 (m, 4H), 2.91-2.83 (m, 1H), 2.79-2.69 (m, 2H), 2.15-2.09 (m, 1H), 1.66 (p, J = 7.4 Hz, 2H), 1.50-1.43 (m, 2H), 1.41-1.28 (m, 17H).

LRMS (ESI) *m*/*z* ([M+H]⁺) cacld for C₅₄H₅₉Cl₂N₇O₉: 1020.4, found: 1020.4.

Preparation Example 2-4-5. Synthesis of Compound 21 (2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-((4-(((2-(2,6-dioxopiperidin-3-yl)-1,3-dioxoisoindolin-4-yl)oxy)methyl)benzyl)oxy)ethyl)acetamide)

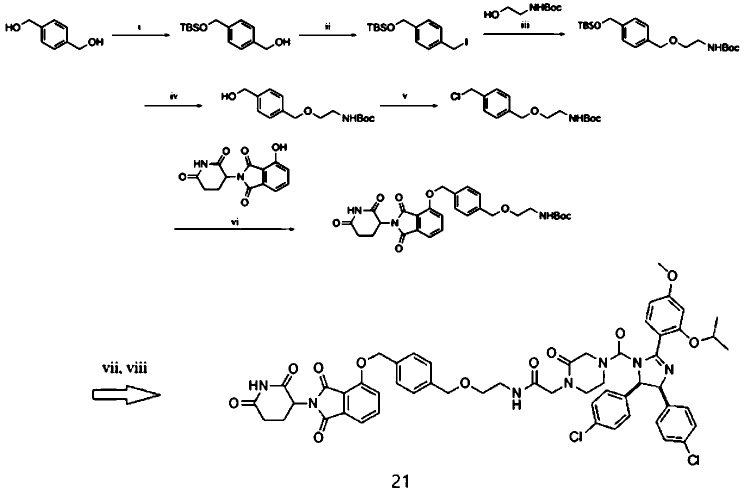

Reagents and conditions: (i) imidazole, TBSCl, THF, 0°C->rt, 12 h; (ii) PPh₃, imidazole, I₂, imidazole, Et 2O/MeCN (3:1), rt, 1h; (iii) TBAI, KOH, THF, rt, 40 h; (iv) TBAF, THF, rt, 3 h; (v) MsCl, DIPEA, DCM, 0°C->rt, 16 h; (vi) NaHCO₃, KI, DMF, 80°C, 16 h; (vii) TFA, CH₂Cl₂, rt, 1 h; (viii) nutlin acid, HATU, DIPEA, DMF, rt, 16 h.

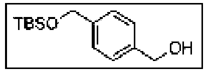

(4-(((tertButyldimethylsilyl)oxy)methyl)phenyl)methanol. TBSCl (0.75 equiv.) was added to a stirred solution of 1,4-phenylenedimethanol (1.00 equiv.) and imidazole (1.00 equiv.) in THF at 0°C and the reaction mixture was stirred at room temperature for 12 hours. After completion of the reaction, the reaction mixture was poured onto brine and extracted with a hexane/ether (4:1) solution. The combined organic layers were dried over a minimum amount of CaCl₂ and concentrated under reduced pressure. The result was purified by column chromatography on silica gel (10% ethyl acetate/hexane) to obtain a product with a yield of 39%.

¹H NMR (600 MHz, CDCl₃) δ 7.35-7.30 (m, 4H), 4.74 (s, 2H), 4.68 (s, 2H), 0.94 (s, 9H), 0.10 (s, 6H).

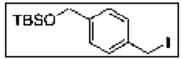

tert-Butyl((4-(iodomethyl)benzyl)oxy)dimethylsilane. PPh₃ (1.10 equiv) and imidazole (2.00 equiv) were dissolved in a 3:1 mixture of Et₂O/ACN. The reaction mixture was stirred until it was completely dissolved. At this time, I₂ was added thereto and vigorous stirring was continued until a yellow suspension was formed. A solution of (4-(((tert-butyldimethylsilyl)oxy)methyl)phenyl)methanol (1.00 equiv) was added to the mixture and stirred for 20 hours. After completion of the reaction, the solvent was removed under vacuum. The mixture was diluted with hexane and washed with saturated NaHSO₄. The result was purified by column chromatography on silica gel (5% ethyl acetate/hexane) to obtain a product with a yield of 47%.

¹H NMR (600 MHz, CDCl₃) δ 7.36-7.33 (m, 2H), 7.25 (d, J = 8.0 Hz, 2H), 4.70 (s, 2H), 4.47 (s, 2H), 0.94 (s, 9H), 0.10 (s, 6H).

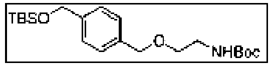

tert-Butyl (2-((4-(((tertbutyldimethylsilyl)oxy)methyl)benzyl)oxy)ethyl)carbamate . TBAI (0.20 equiv.) and KOH (2.00 equiv.) were added to a stirred solution of tert-butyl((4-(iodomethyl)benzyl)oxy)dimethylsilane (1.00 equiv.) and tert-butyl (2-hydroxyethyl)carbamate (1.50 equiv.) in anhydrous THF. The reaction mixture was stirred at room temperature for 40 hours. The solvent was removed under reduced pressure. The result was purified by column chromatography on silica gel (10% ethyl acetate/hexanes) to obtain a product with a yield of 83%.

¹H NMR (600 MHz, CDCl₃) δ 7.32-7.27 (m, 4H), 4.90 (s, 1H), 4.73 (s, 2H), 4.50 (s, 2H), 3.53 (t, J = 5.1 Hz, 2H), 3.37-3.28 (m, 2H), 1.44 (s, 9H), 0.94 (s, 9H), 0.09 (s, 6H).

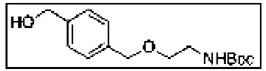

tert-Butyl (2-((4-(hydroxymethyl)benzyl)oxy)ethyl)carbamate. A stirred solution of tert-butyl (2-((4-(((tertbutyldimethylsilyl)oxy)methyl)benzyl)oxy)ethyl)carbamate (1.00 equiv) in anhydrous THF was cooled to 0°C and TBAF (1 M in THF) (1.20 equiv) was added dropwise thereto. The reaction mixture was stirred at 0°C for 2 hours. After completion of the reaction, the reaction was quenched with aqueous NH₄Cl and extracted with ethyl acetate. The result was purified by column chromatography on silica gel (10%→30% ethyl acetate/hexane) to obtain a product with a yield of 82%.

¹H NMR (600 MHz, CDCl₃) δ 7.39-7.31 (m, 4H), 4.90 (s, 1H), 4.70 (s, 2H), 4.52 (s, 2H), 3.54 (t, J = 5.2 Hz, 2H), 3.34 (d, J = 5.5 Hz, 2H), 1.44 (s, 9H).

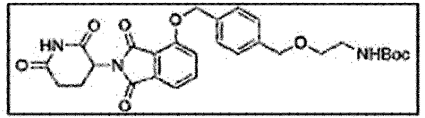

tert-Butyl (2-((4-(chloromethyl)benzyl)oxy)ethyl)carbamate. MsCl (2.00 equiv) was added dropwise to a stirred solution of tert-butyl (2-((4-(hydroxymethyl)benzyl)oxy)ethyl)carbamate (1.00 equiv) and DIPEA (0.20 equiv) in CH₂Cl₂ at 0°C. The reaction mixture was stirred at 0°C for 1 hour and warmed to room temperature for 16 hours. The result was purified by column chromatography on silica gel (10% ethyl acetate/hexanes) to obtain a product with a yield of 95%.

¹H NMR (600 MHz, CDCl₃) δ 7.40 - 7.35 (m, 2H), 7.32 (d, J = 8.1 Hz, 2H), 4.89 (s, 1H), 4.59 (s, 2H), 4.52 (s, 2H), 3.54 (t, J = 5.2 Hz, 2H), 3.35 (d, J = 6.1 Hz, 2H), 1.44 (s, 9H).

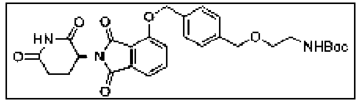

tert-Butyl (2-((4-(((2-(2,6-dioxopiperidin-3-yl)-1,3-dioxoisoindolin-4-yl)oxy)methyl)benzyl)oxy)ethyl)carbamate. NaHCO₃ (1.50 equiv) and KI (1.00 equiv) were added to a stirred solution of 2-(2,6-dioxopiperidin-3-yl)-4-hydroxyisoindoline-1,3-dione (1.10 equiv) in anhydrous DMF. The reaction mixture was stirred at 80°C for 16 hours. The result was purified by column chromatography on silica gel (40%-60% ethyl acetate/hexane) to obtain a product with a yield of 57%.

¹H NMR (600 MHz, CDCl₃) δ 8.21 (s, 1H), 7.63 (ddd, J = 8.5, 7.1, 1.2 Hz, 1H), 7.50-7.42 (m, 3H), 7.35 (d, J = 7.9 Hz, 2H), 7.28-7.19 (m, 2H), 4.97 (dd, J = 12.5, 5.4 Hz, 1H), 4.90 (d, J = 17.2 Hz, 1H), 4.51 (s, 2H), 3.54 (t, J = 5.1 Hz, 2H), 3.34 (s, 2H), 2.92-2.70 (m, 3H), 2.19-2.08 (m, 1H), 1.43 (s, 10H).

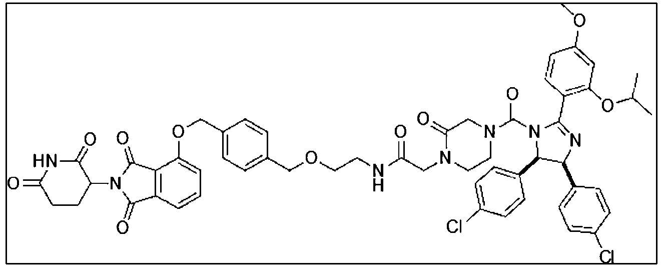

2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-((4-(((2-(2,6-dioxopiperidin-3-yl)-1,3-dioxoisoindolin-4-yl)oxy)methyl)benzyl)oxy)ethyl)acetamide. The title compound was synthesized using General Method C and then General Method D, and was obtained as a white solid with a yield of 22%.

¹H NMR (600 MHz, CD₃OD) δ = 7.71 (ddd, J =8.4, 7.4, 0.9, 1H), 7.54 (t, J =8.0 Hz, 1H), 7.49 (d, J =7.3 Hz, 2H), 7.45 (dd, J =8.5, 2.4 Hz, 1H), 7.43 (d, J =7.3 Hz, 1H), 7.35 (d, J =8.1 Hz, 2H), 7.12 (d, J =8.4 Hz, 2H), 7.06 (dd, J =8.8, 1.7 Hz, 2H), 7.00 (d, J =8.4 Hz, 2H), 6.91 (d, J =7.6 Hz, 2H), 6.64-6.61 (m, 2H), 5.74 (d, J =8.0 Hz, 1H), 5.56 (dd, J =10.3, 3.2 Hz, 1H), 5.32 (s, 2H), 5.11-5.06 (m, 1H), 4.71 (dd, J =12.2, 6.0 Hz, 1H), 4.49 (s, 2H), 3.91 (dd, J =16.2, 8.5 Hz, 1H), 3.84 (d, J =2.6 Hz, 3H), 3.83-3.71 (m, 3H), 3.51 (t, J =5.5 Hz, 2H), 3.49-3.44 (m, 1H), 3.41-3.30 (m, 5H), 2.97-2.92 (m, 2H), 2.86-2.78 (m, 1H), 2.74-2.64 (m, 2H), 2.10-2.04 (m, 1H), 1.34 (d, J =6.0 Hz, 3H), 1.31 (dd, J =6.0, 3.3 Hz, 3H).

LRMS (ESI) *m*/*z* ([M+H]⁺) calcd for C₅₅H₅₃Cl₂N₇O₁₁: 1058.3, found 1058.3

### Example 1: Development of MDM2-targeting PROTACs using CRBN ligand

Human dental pulp stem cell lines (hDPSCs) were treated with 14 types of synthesized MDM2-PROTACs (10 µM) for 1 hour and Western blot analysis using SDS-PAGE gel was performed to evaluate the intracellular MDM2 protein degradation capacity.

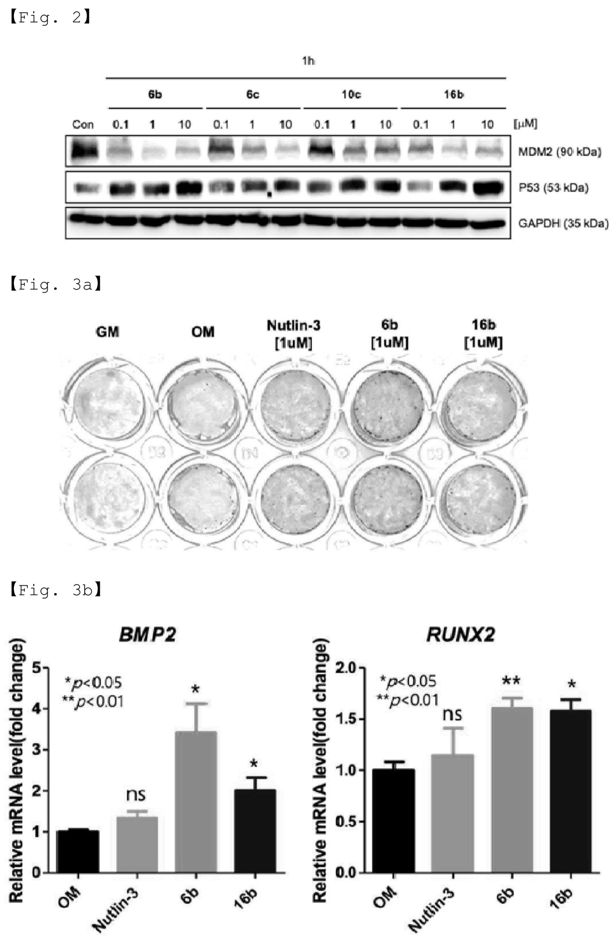

As can be seen from FIGS. 1A and 1B, MDM2 was degraded. As shown in FIG. 2, based on the initially selected compounds, two compounds (compounds 6b and 16b) and treatment concentrations that effectively degrade MDM2 and activate p53, and treatment concentrations were selected.

### Example 2: Induction of Osteogenic Differentiation

Human-derived dental pulp stem cell lines (hDPSCs) were treated with the secondarily selected compounds, i.e., Compound 6b, Compound 16b, and Compounds 17 to 21, at a concentraiton of 1 µM for 2 weeks, and then immobilized with 4% PFA, and the degree of osteoblast differentiation was assessed by Alizarin Red S staining. Human-derived dental pulp stem cell lines (hDPSCs) were treated with the secondarily selected compounds, i.e., Compound 6b, Compound 16b, and Compounds 17 to 21, at a concentraiton of 1 µM for 2 weeks. Quantitative PCR was then performed to determine the genetic changes in the osteogenic differentiation markers, i.e., BMP2 and RUNX2.

FIGS. 3A and 3B show the biomineralization effect (A) of the secondarily selected compounds confirmed by Alizarin Red S staining and the expression of osteogenic differentiation marker genes.

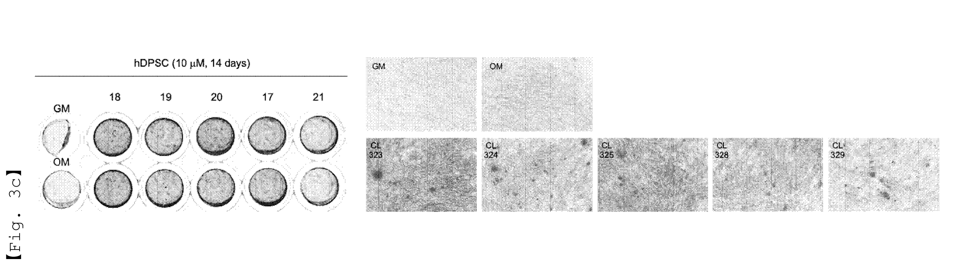

FIG. 3C shows the biomineralization effect of compounds 17 to 21 confirmed by Alizarin Red S staining.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

### [Industrial Applicability]

The novel nutlin-based compound according to the present invention effectively promotes differentiation of mesenchymal stromal cells by inducing degradation of MDM2, a negative regulator of p53, and thus has an excellent effect in inducing osteogenic differentiation of stem cells.

Although specific configurations of the present invention have been described in detail, those skilled in the art will appreciate that this detailed description is provided as preferred embodiments for illustrative purposes and should not be construed as limiting the scope of the present invention. Therefore, the substantial scope of the present invention is defined by the accompanying filed claims and equivalents thereto.

## Claims

1. A compound of Formula (I), a stereoisomer thereof, or a pharmaceutically acceptable salt thereof: 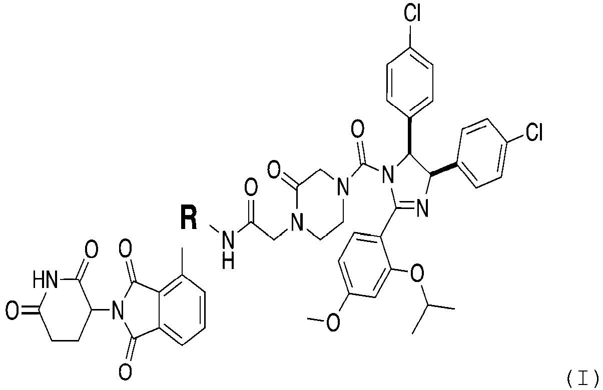
wherein R is 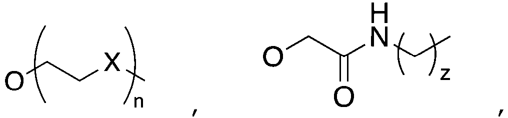 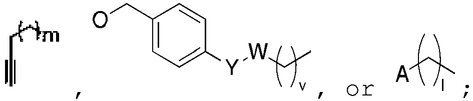
X is C, O, or N, n is an integer of 1 to 5;
Z is an integer of 1 to 10;
m is an integer of 1 to 10;
Y-W is C≡C, or both Y and W are C, or Y is C and W is O;
v is an integer of 1 to 4;
A is C or N; and
l is an integer of 1 to 8.

2. The compound, the stereoisomer thereof, or the pharmaceutically acceptable salt thereof according to claim 1, wherein
R is 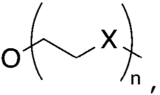
X is C or O, and
n is an integer of 1 to 3.

3. The compound, the stereoisomer thereof, or the pharmaceutically acceptable salt thereof according to claim 1, wherein
R is 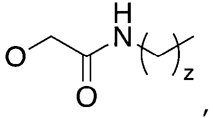 and
Z is an integer of 2 to 8.

4. The compound, the stereoisomer thereof, or the pharmaceutically acceptable salt thereof according to claim 1, wherein
R is 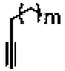 and
m an integer of 3 to 9.

5. The compound, the stereoisomer thereof, or the pharmaceutically acceptable salt thereof according to claim 1, wherein
R is 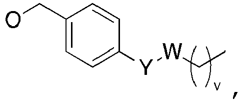
Y-W is C≡C, or both Y and W are C, or Y is C and W is O, and
v is an integer of 1 to 3.

6. The compound, the stereoisomer thereof, or the pharmaceutically acceptable salt thereof according to claim 1, wherein
R is 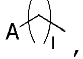
A is C or N, and
l is an integer of 1 to 4.

7. The compound, the stereoisomer thereof, or the pharmaceutically acceptable salt thereof according to claim 1, wherein the compound comprises at least one selected from the group consisting of:
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(5-((2-(2,6dioxopiperidin-3-yl)-1,3-dioxoisoindolin-4-yl)oxy)pentyl) acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(8-((2-(2,6-dioxo-opiperidin-3-yl)-1,3-dioxoisoindolin-4-yl)oxy)octyl) acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(11-((2-(2,6-dioxop-erydin-3-yl)-1,3-dioxoisoindolin-4-yl)oxy)undecyl) acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-((2-(2,6-dioxopiperidin-3-yl)-1,3-dioxoisoindolin-4-yl)oxy) ethoxy)ethyl)acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-(2-((2-(2,6-dioxo-opiperidin-3-yl))-1,3-dioxoisoindolin-) 4-yl)oxy)ethoxy)ethoxy)ethoyl)acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-(2-(2-((2-(2,6-dioxopiperidin-3-yl))-1,3-dioxo)isoindolin-4-yl)oxy))ethoxy)ethoxy)-ethoxy)ethyl)-acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-(2-((2-(2,6-dioxopiperidin-3-yl)-1,3-dioxoisoindolin-4-yl))oxy)acetamide)ethyl)-acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(5-(2-((2-(2,6-dioxopiperidin-3-yl)-1,3-dioxoisoindolin-4-yl))oxy)acetamide)pentyl-l)acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(8-(2-((2-(2,6-dioxopiperidin-3-yl)-1,3-dioxoisoindolin-4-yl))oxy)acetamide)octyl)-acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(6-(2-(2,6-dioxopipe-lysin-3-yl)-1,3-dioxoisoindolin-4-yl)hex-5-yn-1-yl) acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(9-(2-(2,6-dioxopiperidin-3-yl)-1,3-dioxoisoindolin-4-yl)non-8-yn-1-yl) acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(9-(2-(2,6-dioxopiperidin-3-yl)-1,3-dioxoisoindolin-4-yl)dodec-11-yn-1-yl) acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(8-((2-(2,6-dioxopiperidin-3-yl)-1,3-dioxoisoindolin-4-yl)amino)octyl)acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(4-(4-(((2-(2,6-dioxopiperidin-3-yl)-1,3-dioxoisoindolin-4-yl)oxy)methyl)phenyl)but-3-yn-1-yl)acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(4-(4-(((2-(2,6-dioxopiperidin-3-yl)-1,3-dioxoisoindolin-4-yl)oxy)methyl)phenyl) butyl)acetamide;
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(9-(2-(2,6-dioxopiperidin-3-yl)-1,3-dioxoisoindolin-4-yl)nonyl)acetamide; and
2-(4-(cis-4,5-bis(4-chlorophenyl)-2-(2-isopropoxy-4-methoxyphenyl)-4,5-dihydro-1H-imidazole-1-carbonyl)-2-oxopiperazin-1-yl)-N-(2-((4-(((2-(2,6-dioxopiperidin-3-yl)-1,3-dioxoisoindolin-4-yl)oxy)methyl)benzyl)oxy)ethyl)acetamide.

8. A composition for inducing osteogenic differentiation of stem cells comprising the compound, the stereoisomer thereof, or the pharmaceutically acceptable salt thereof according to any one of claims 1 to 7.

9. The composition according to claim 8, wherein the composition is prepared into any one formulation selected from the group consisting of injections, oral preparations, patches, liquids, capsules, granules, tablets, powders, sprays, ointments, gels, mucosal preparations, and suppositories.

10. The composition according to claim 8, furthe r comprising a pharmaceutically acceptable carrier.

11. The composition according to claim 8, wherein a daily dose of the compound, the stereoisomer thereof, or the pharmaceutically acceptable salt thereof is 1 ug/kg to 100 mg/kg, and is administered once daily or 2 to 3 times a week.
